# EUROPEAN PATENT APPLICATION

(11) **EP 3 216 788 A1**
(43) Date of publication of application: **13.09.2017**
(21) Application number: 16159846.1
(22) Date of filing: 11.03.2016
(51) Int. Cl.: C07D 403/06

(54) **ALPHA-HYDROXY-BETA-TRIAZOLO-TETRAZOLES**

(71) Applicant: Centre National de la Recherche Scientifique, 75016 Paris (FR); Université Versailles Saint-Quentin, 78035 Versailles Cedex (FR)
(72) Inventor: WRIGHT, Karen, 92210 Saint-Cloud (FR); COUTY, François, 92240 Malakoff (FR); QUINODOZ, Pierre, 74160 Saint-Julien-en-Genevois (FR); DROUILLAT, Bruno, 92210 Saint-Cloud (FR)
(74) Representative: Icosa

(57) **Abstract**

The present invention relates to alpha-hydroxy-beta-triazolo-tetrazoles of formula (I):

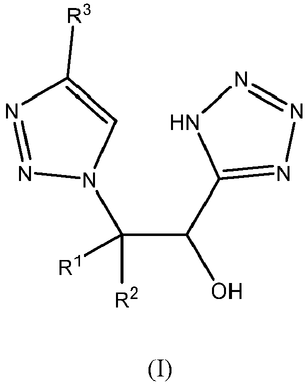

The present invention also relates to a process for manufacturing alpha-hydroxy-beta-triazolo-tetrazoles of formula (I).

The present invention further relates to the use of alpha-hydroxy-beta-triazolo-tetrazoles for synthesizing new compounds, e.g. in "click" chemistry.

## Description

### FIELD OF INVENTION

The present invention pertains to chemistry, especially to organic chemistry and to the synthesis of organic compounds.

The present invention relates to alpha-hydroxy-beta-triazolo-tetrazole compounds and their manufacturing process. The present invention further relates to the use of alpha-hydroxy-beta-triazolo-tetrazoles for synthesizing new compounds, especially but not limitatively in "click" chemistry.

### BACKGROUND OF INVENTION

Tetrazoles and their derivatives have found applications in various domains including material science, energetic materials, coordination chemistry, organic synthesis, and especially medicinal chemistry, due to the fact that 5-substituted tetrazoles (5-ST) are bioisosteres of carboxylic acids. Therefore, there is a need of novel methods for introducing a tetrazole functional group in compounds, e.g. in organic molecules.

Triazoles and their derivatives are well-known as antifungal agents, and are also convenient intermediates for chemical synthesis. Moreover, since the triazole heterocycle is an analogue of the peptide bound, non-peptidic polymeric compounds comprising triazole groups may form peptide-like chains (Angelo, N. G. et al., Journal of the America Chemical Society, 2005, Vol. 127, No. 49, pp.17134-17135.). Such biomimetic molecules may lead to substantive progress in biotechnologies, especially in medicinal chemistry.

The 1,3-dipolar cycloaddition (Huisgen reaction) of azides and alkynes leads to five-membered triazole heterocycles and has gained considerable interest in the field of organic synthesis since the development of cooper (I)-catalysed procedures by Meldal and Tornøe. Copper-catalysed azide-alkyne cycloaddition (CuAAC) is a well-known "click" reaction, which is very general and has many attractive features, including: high or quantitative yields, robustness, insensibility, orthogonality, and compatibly with biological and polymerization conditions (Meldal, M. and Tornøe, C. W., Chemical Reviews 2008, Vol. 108, pp. 2952-3015.). A particularly advantageous aspect of CuAAC is that it allows orthogonal ligation reactions, which means that a dedicated set of reaction conditions will lead to a ligation reaction occurring specifically on a functional group of a molecule, without affecting the others.

Due to the popularity of CuAAC reaction, many libraries of compatible azides and alkynes are available. However, there is a need for improvement of the selectivity of CuAAC reactions and/or reduction of the number of steps for the synthesis of complex molecules. Therefore, there is still a need for novel reactants having specific features advantageous for CuAAC and presenting a variety of reactive groups, especially tetrazole groups.

There is also a need for reactants comprising "latent" or "hidden" functional groups, i.e. groups which will not react in CuAAC conditions, but may be easily converted to azide or alkyne when another reactive group is required, thus allowing sequential CuAAC reactions.

The applicant surprisingly established that alpha-hydroxy-beta-triazolo-tetrazoles could be useful molecular scaffolds for chemical synthesis, in particular for sequenced synthesis.

Therefore, the applicant conceived and successfully achieved the manufacture of alpha-hydroxy-beta-triazolo-tetrazoles and their use as reactants, especially in "click" reactions such as CuAAC.

### SUMMARY

In a first aspect, the invention relates to an alpha-hydroxy-beta-triazolo-tetrazole compound of formula (I): 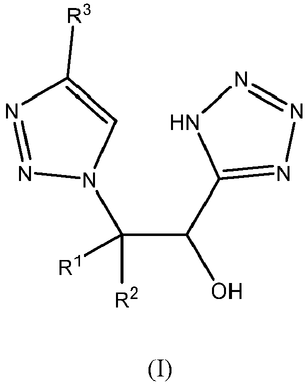 wherein R¹ and R² are each independently hydrogen, hydrocarbyl, aryl, heteroaryl, hydrocarbylaryl, arylhydrocarbyl, hydrocarbylheteroaryl, or heteroarylhydrocarbyl groups; or R¹ and R² form together a group being hydrocarbyl, aryl, heteroaryl, hydrocarbylaryl, arylhydrocarbyl, hydrocarbylheteroaryl, or heteroarylhydrocarbyl;
wherein the group is optionally substituted by at least one group being hydrocarbyl, heteroaryl, oxo, hydroxyl, amido, amino, nitro, carboxylo, formyl, halo, thioxo or sulfhydryl;
wherein the group is optionally interrupted or terminated by at least one group being -O-; -S-; or -NR^{N}- wherein R^{N} is hydrogen, hydrocarbyl, aryl, or a combination thereof;
wherein the nitrogen or sulfur atoms substituting or comprised in the group are optionally oxidized; and
wherein R³ is hydrogen, an organic group or an organic molecule;
and stereoisomers thereof; and salts thereof; and solvates thereof.

According to an embodiment, R¹ and R² are each hydrogen, alkyl, alkenyl, aryl, heteroaryl, alkylaryl, arylalkyl, alkenylaryl, or arylalkenyl groups; or R¹ and R² form together a group being alkyl, alkenyl, aryl, alkylaryl, arylalkyl, alkenylaryl or arylalkenyl; the group is optionally substituted by at least one group being hydroxyl, alkyl, alkenyl, aryl, alkylaryl, arylalkyl, amino, nitro, halo or sulfhydryl; and the group is optionally interrupted or terminated by at least one group being -O-; -S-; or -NR^{N}- with R^{N} is hydrogen, alkyl, alkenyl, aryl, or a combination thereof.

According to a specific embodiment, R¹ and R² are each independently hydrogen, alkyl, aryl or alkenylaryl groups; or R¹ and R² form together a group being alkyl and aryl; wherein the group is optionally substituted by at least one halo group.

According to an embodiment, R³ is hydrogen, hydroxyl, amido, amino, cyano, tetrazolyl, triazolyl, nitro, borono, carboxylo, formyl, halo, haloformyl, phosphono, phosphate or sulfhydryl;
or R³ is a hydrocarbyl, aryl, heteroaryl, hydrocarbylaryl, arylhydrocarbyl, hydrocarbylheteroaryl, or heteroarylhydrocarbyl group;
wherein the group is optionally substituted by at least one group being hydrocarbyl, aryl, heteroaryl, oxo, hydroxyl, amido, amino, cyano, tetrazolyl, triazolyl, nitro, carboxylo, formyl, halo, thioxo or sulfhydryl;
wherein the group is optionally interrupted or terminated by at least one group being -O-; -S-; or -NR^{N}- wherein R^{N} is hydrogen, hydrocarbyl, aryl, or a combination thereof; and
wherein the nitrogen or sulfur atoms substituting or comprised in the group are optionally oxidized;
or R³ is a carbohydrate, an amino acid, a peptide or a nucleoside.

According to a specific embodiment, R³ is an alkyl, alkenyl, aryl, heteroaryl, alkylaryl, arylalkyl, alkenylaryl, arylalkenyl, alkylheteroaryl, or heteroarylalkyl group; wherein the group is optionally substituted by at least one group being alkyl, alkenyl, aryl, heteroaryl, alkylaryl, arylalkyl, oxo, hydroxyl, amido, amino, tetrazolyl, triazolyl, nitro, carboxylo, formyl, halo, thioxo or sulfhydryl; and the group is optionally interrupted or terminated by at least one group being -O-; -S-; or -NR^{N}- wherein R^{N} is hydrogen, alkyl, alkenyl, aryl, or a combination thereof.

According to a more specific embodiment, the compound is selected from the group consisting of: (1-(4-phenyl-1H-1,2,3-triazol-1-yl)cyclohexyl)(1H-tetrazol-5-yl)methanol; (1-(4-phenyl-1H-1,2,3-triazol-1-yl)cycloheptyl)(1H-tetrazol-5-yl)methanol; (1-(4-phenyl-1H-1,2,3-triazol-1-yl)cyclooctyl)(1H-tetrazol-5-yl)methanol; tert-butyl 1-(1-(hydroxy(1H-tetrazol-5-yl)methyl)cyclooctyl)-1H-1,2,3-triazole-4-carboxylate; (1-(4-(3-chloropropyl)-1H-1,2,3-triazol-1-yl)cyclooctyl)(1H-tetrazol-5-yl)methanol; 2-(1-(1-(hydroxy(1H-tetrazol-5-yl)methyl)cyclooctyl)-1H-1,2,3-triazol-4-yl)ethan-1-ol; 2-ethyl-2-(4-phenyl-1H-1,2,3-triazol-1-yl)-1-(1H-tetrazol-5-yl)butan-1-ol; 2-(4-hexyl-1H-1,2,3-triazol-1-yl)-2-phenyl-1-(1H-tetrazol-5-yl)ethan-1-ol; 2-(4-hexyl-1H-1,2,3-triazol-1-yl)-2,2-diphenyl-1-(1H-tetrazol-5-yl)ethan-1-ol; 2-(4-(3-chloropropyl)-1H-1,2,3-triazol-1-yl)-2,2-diphenyl-1-(1H-tetrazol-5-yl)ethan-1-ol and tert-butyl 1-(2-hydroxy-1,1-diphenyl-2-(1H-tetrazol-5-yl)ethyl)-1H-1,2,3-triazole-4-carboxylate.

In a second aspect, the invention relates to a process for manufacturing an alpha-hydroxy-beta-triazolo-tetrazole of formula (I): 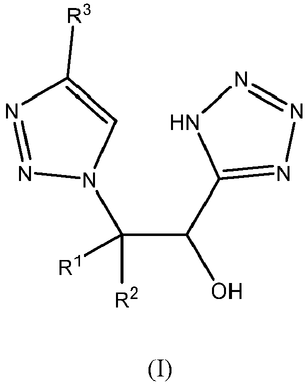 wherein R¹ and R² are each independently hydrogen, hydrocarbyl, aryl, heteroaryl, hydrocarbylaryl, arylhydrocarbyl, hydrocarbylheteroaryl, or heteroarylhydrocarbyl; or wherein R¹ and R² form together a group being hydrocarbyl, aryl, heteroaryl, hydrocarbylaryl, arylhydrocarbyl, hydrocarbylheteroaryl, or heteroarylhydrocarbyl;
wherein the group is optionally substituted by at least one group being hydrocarbyl, aryl, heteroaryl, oxo, hydroxyl, amido, amino, nitro, carboxylo, formyl, halo, thioxo or sulfhydryl;
wherein the group is optionally interrupted or terminated by at least one group being -O-; -S-; or -NR^{N}- wherein R^{N} is hydrogen, hydrocarbyl, aryl, or a combination thereof; and
wherein the nitrogen or sulfur atoms are optionally oxidized; and
wherein R³ is hydrogen, an organic group or an organic molecule;
comprising starting from an alpha hydroxy-beta-azido-tetrazole of formula (II): 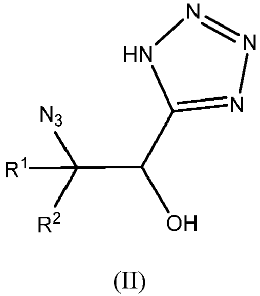 and a terminal alkyne of formula R³-C≡C-H, and
carrying out the reaction of compound (II) with alkyne R³-C≡C-H in the presence of a copper(I) source and a tertiary amine,
wherein the copper(I) source is either:
- a combination of a copper(I) salt and a base, or
- a combination of a copper(II) salt and a reducing agent.

According to an embodiment, the copper(I) source is a combination of a copper(I) salt being copper(I) chloride, copper(I) bromide or copper(I) acetate; preferably copper(I) bromide; and a base being N,N-diisopropylethylamine.

According to an embodiment, the copper(I) source is a combination of a copper(II) salt being copper(II) chloride, copper(II) bromide, copper(II) acetate or copper(II) sulphate; preferably copper(II) sulphate; and a reducing agent being sodium ascorbate or tri(2 carboxyethyl)phosphine; preferably sodium ascorbate; and the copper salt/reducing agent molar ratio in the reaction medium ranges from 0.1 to 2; preferably from 0.25 to 0.75; more preferably is 0.5.

According to an embodiment, the tertiary amine is tris(benzyltriazolylmethyl)amine, tris(tertbutyltriazolylmethyl)amine, tris(benzimidazole)methyl amine , 4,7-diphenyl-1,10-phenanthroline-disulfonic acid disodium salt, tris[2 (N,N dibenzylamino)ethyl]amine or tris(benzyltriazolylmethyl)amine; preferably tris(benzyltriazolylmethyl)amine.

According to an embodiment, the alkyne/azide molar ratio in the reaction medium ranges from 1 to 10, preferably from 2 to 5, more preferably is 3; and the copper salt/azide molar ratio in the reaction medium ranges from 0.01 to 2, preferably from 0.05 to 0.2, more preferably is 0.1.

According to an embodiment, the copper salt/tertiary amine molar ratio in the reaction medium ranges from 0.1 to 10; preferably from 0.5 to 2; preferably is 1.

According to an embodiment, the reaction is executed in a solvent; preferably ethanol, tetrahydrofuran, N,N'-dimethylformamide, acetonitrile, n-butyl alcohol, water or mixtures thereof; more preferably a mixture of n-butyl alcohol and water.

According to an embodiment, the reaction is executed in a duration ranging from 12h to 5 days; preferably from 24h to 72h; more preferably for 48h.

According to an embodiment, the reaction is executed at a temperature ranging from 0 to 50°C; preferably from 15 to 30°C; more preferably at 25°C.

In others aspects, the invention relates to a triazole alkyne of formula (III): 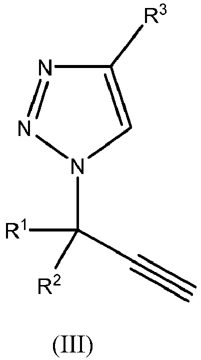 wherein R¹ and R² are each independent groups as previously disclosed; or R¹ and R² form together a group as previously disclosed; and R³ is a group as previously disclosed; and stereoisomers thereof; and salts thereof; and solvates thereof.

In others aspects, the invention relates to reactions, especially CuAAC reactions, wherein compounds (I) and/or (III) are used as reactants.

### DEFINITIONS

In the present invention, the following terms have the following meanings:
- **"about"** preceding a figure means plus or less 10% of the value of said figure.
- **"alkyl"** refers to a linear, cyclic or branched saturated hydrocarbon chain of general formula -CₙH₂ₙ₊₁ wherein n is a number greater than or equal to 1, typically containing 1 to 16 carbon atoms, preferably 1 to 12 carbon atoms, more preferably 1 to 8 carbon atoms. When concerning a group being bounded twice to the same carbon atom, "alkyl" also refers to an alkylenyl derived from an alkyl by removal of a hydrogen atom. Examples of alkyl groups are methyl, ethyl, *n*-propyl, isopropyl, butyl, pentyl, hexyl, cyclopentyl and 2-ethylcyclohexyl.
- **"alkenyl"** refers to a linear, cyclic or branched unsaturated hydrocarbon chain, typically containing 1 to 16 carbon atoms, preferably 1 to 12 carbon atoms, more preferably 1 to 8 carbon atoms, wherein the unsaturation arises from the presence of one or more carbon-carbon double bonds. When concerning a group being bounded twice to the same carbon atom, "alkenyl" also refers to an alkenylenyl derived from an alkenyl by removal of a hydrogen atom. Examples of alkenyl groups are propenyl, butenyl, hexenyl and 3-ethylcyclohex-2-enyl.
- **"alkynyl"** refers to linear, cyclic or branched unsaturated hydrocarbon chain, typically containing 1 to 16 carbon atoms, preferably 1 to 12 carbon atoms, more preferably 1 to 8 carbon atoms, wherein the unsaturation arises from the presence of one or more carbon-carbon triple bonds. When concerning a group being bounded twice to the same carbon atom, "alkynyl" also refers to an alkynylenyl derived from an alkynyl by removal of a hydrogen atom. Examples of alkynyl groups are propynyl, butynyl, hexynyl and 3-ethylcyclohex-2-ynyl.
- **"amido"** refers to -C(=O)-NH₂ group.
- **"amino"** refers to -NH₂ group.
- "**aryl**" refers to a polyunsaturated, aromatic hydrocarbon chain having a single ring (i.e. phenyl) or multiple rings fused together (e.g. naphtyl) or linked covalently, wherein at least one ring is aromatic, typically containing 5 to 16 carbon atoms, preferably 5 to 10 carbon atoms, more preferably 5 or 6 carbon atoms. "**Aryl**" also refers to the partially hydrogenated derivatives of the carbocyclic systems enumerated herein. Examples of aryl comprise phenyl, biphenylyl, biphenylenyl, 5- or 6-tetralinyl, naphthalen-1- or -2-yl, 4-, 5-, 6 or 7-indenyl, 1- 2-, 3-, 4- or 5-acenaphtylenyl, 3-, 4- or 5-acenaphtenyl, 1- or 2-pentalenyl, 4- or 5-indanyl, 5-, 6- , 7- or 8-tetrahydronaphthyl, 1,2,3,4-tetrahydronaphthyl, 1,4-dihydronaphthyl, 1-, 2-, 3-, 4- or 5-pyrenyl.
- "**azide**" refers to a molecule comprising the azido group.
- **"azido"** refers to -N₃ (-N=N⁽⁺⁾=N⁽⁻⁾) group.
- **"borono"** refers to -B(OH)₂ group.
- **"carboxylo"** refers to carboxylic acid -COOH group.
- "**cyano**" or **"carbonitrile"** refers to -C≡N group.
- **"cycloalkyl", "cycloalkenyl"** and **"cycloalkynyl"** respectively refers to a cyclic or polycyclic alkyl, alkenyl and alkynyl group, typically containing 5 to 16 atoms, optionally branched. Non-limiting examples of cycloalkyl are cyclopropyl, cyclopentyl and cyclohexyl.
- **"hydrocarbyl"** refers to any alkyl, alkenyl or alkynyl group as defined above. Unless otherwise stated, any term defined in this section which include this term "hydrocarbyl" also define every corresponding term wherein "hydrocarbyl" is substituted by "alkyl", "alkenyl" or "alkynyl". For example, "halohydrocarbyl" as defined hereafter define simultaneously by similarity "haloalkyl", "haloalkenyl" or "haloalkynyl".
- **"heteroaryl"** refers to an aryl group as defined above, wherein one or more carbon atoms in one or more aromatic rings are replaced by oxygen, nitrogen or sulfur atoms, and where the nitrogen and sulfur heteroatoms may optionally be oxidized and the nitrogen heteroatoms may optionally be quaternized. Examples of such heteroaryl are pyrrolyl, furanyl, thiophenyl and pyrazolyl.
- **"hydrocarbylaryl"** and **"arylhydrocarbyl"** respectively refers to an aryl group substituted by, or fused with, a hydrocarbyl group and to a hydrocarbyl group substituted by, or fused with, an aryl group.
- **"aryloxyl"** refers to -O-aryl group.
- **"hydrocarbyloxyl"** refers to -O-hydrocarbyl group.
- **"hydrocarbylheteroaryl"** and **"heteroarylhydrocarbyl"** respectively refers to a heteroaryl group substituted by, or fused with, a hydrocarbyl group and to a hydrocarbyl group substituted by, or fused with, a heteroaryl group.
- **"haloaryl"** refers to an aryl group as defined above, further comprising at least one halo group.
- **"halohydrocarbyl"** refers to a hydrocarbyl group as defined above, further comprising at least one halo group.
- **"formyl"** refers to aldehyde -CHO group.
- **"halo"** refers to fluoride, chloride, bromide or iodide atoms.
- **"haloformyl"** refers to -C(=O)X group, wherein X is halo group as defined above.
- **"hydroxyl"** refers to -OH group.
- **"nitro"** refers to -NO₂ group.
- **"nitrile"** refers to a molecule comprising the cyano group.
- **"oxo"** refers to =O group, i.e. an oxygen atom forming a double bond with a carbon atom.
- **"phosphono"** refers to -P(=O)(OH)₂ group.
- **"phosphato"** refers to -O-P(=O)(OH)₂ group.
- **"prodrug"** refers to derivatives of a biologically active drug compound, such as for example amides, whose *in vivo* biotransformation product generates the biologically active drug. Prodrugs are generally characterized by increased bio-availability and are readily metabolized into biologically active compounds *in vivo.*
- **"protective group"** refers to a functional group that masks the characteristic reactivity of another group to which it can later be converted. Examples of protecting groups are acetyl (Ac), benzoyl (Bz), *tert*-butyloxycarbonyl (BOC), carbobenzoyloxy (Cbz), *p*-methoxybenzyl ether (PMB), silyl ethers such as trimethylsilyl (TMS), *tert*-butyldimethylsilyl (TBDMS) and tetrahydropyranyl (THP).
- **"solvate"** refers to a compound that contains stoichiometric or sub-stoichiometric amounts of one or more solvent molecule such as ethanol. The term "hydrate" refers to when the said solvent is water.
- **"sulfhydryl"** refers to -SH group.
- **"tetrazole"** refers to a molecule comprising the tetrazolo group.
- **"tetrazolo"** refers to a 5-member heterocyclic group, consisting of a ring of four nitrogen and one carbon atom of general formula CRR'N₄, wherein R and R' are either hydrogen or other groups.
- **"tetrazolyl"** refers to the 5-member heterocyclic group -CHN₄.
- **"triazole"** refers to a molecule comprising the triazolo group.
- **"triazolo"** refers to a 5-member heterocyclic group, consisting of a ring of three nitrogen and two carbon atom, of general formula C₂RR'R"N₃. , wherein R, R' and R" are either hydrogen or other groups.
- **"triazolyl"** refers to the 5-member heterocyclic group -CH₂N₃.
- **"thioxo"** refers to =S group, i.e. a sulfur atom forming a double bond with a carbon atom.

In the present invention, the following abbreviations have the following meanings:
- **"Ac"** refers to acetyl group -C(=O)CH₃.
- **"Bu₂SnO"** refers to dibutyltin oxide (C₄H₉)₂Sn=O [No. CAS: 818-08-6].
- "**CuAAC**" refers to a copper-catalysed azide-alkyne cycloaddition reaction.
- "**EDC**" refers to 1-ethyl-3-(3-dimethylaminopropyl)carbodiimide [No. CAS: 1892-57-5].
- **"Et"** refers to ethyl -C₂H₅.
- "**EtOAc**" refers to ethyl acetate [No. CAS: 141-78-6].
- "**DCC**" refers to N,N'-dicyclohexylcarbodiimide [No. CAS: 538-75-0].
- **"DIC"** refers to N, N'-diisopropylcarbodiimide [No. CAS: 693-13-0].
- **"NaAs"** refers to sodium ascorbate [No. CAS: 134-03-2 (L)].
- "**Ph**" refers to phenyl -C₆H₅.
- "**TBTA**" refers to tris(benzyltriazolylmethyl)amine [No. CAS: 510758-28-8].
- **"TMSN₃"** refers to trimethylsilyl azide (CH₃)₃SiN₃ [No. CAS: 4648-54-8].

### DETAILED DESCRIPTION

### Alpha-hydroxy-beta-triazolo-tetrazoles

In its first aspect, the invention relates to an alpha-hydroxy-beta-triazolo-tetrazole of formula (I): 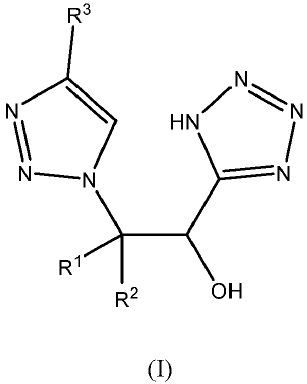 wherein R¹ and R² are each independently hydrogen, hydrocarbyl, aryl, heteroaryl, hydrocarbylaryl, arylhydrocarbyl, hydrocarbylheteroaryl, or heteroarylhydrocarbyl groups;
or
R¹ and R² form together a group being hydrocarbyl, aryl, heteroaryl, hydrocarbylaryl, arylhydrocarbyl, hydrocarbylheteroaryl, or heteroarylhydrocarbyl; and
the group is optionally substituted by at least one group being hydrocarbyl, aryl, heteroaryl, oxo, hydroxyl, amido, amino, azido, cyano, nitro, borono, carboxylo, formyl, halo, haloformyl, phosphono, phosphato, thioxo or sulfhydryl; and
the group is optionally interrupted or terminated by at least one group being -B(OR^{B})- with R^{B} being hydrogen, hydrocarbyl, aryl or a combination thereof; -O-; -PR^{P}- with R^{P} being hydrogen, hydrocarbyl, aryl or a combination thereof; -P(OR^{OP})- with R^{OP} being hydrogen, hydrocarbyl, aryl or a combination thereof; -S-; or -NR^{N}- with R^{N} being hydrogen, hydrocarbyl, aryl or a combination thereof; or a combination thereof;
the nitrogen, phosphorus or sulfur atoms substituting or comprised in the group are optionally oxidized; and
R³ is hydrogen; an organic group such as alkyl, hydroxyl or amino; an organic molecule such as a polymer, a carbohydrate, a protein, an amino acid, a peptide, a nucleoside; an inorganic compound such as a metal salt; or an organometallic compound such as a metal complex.

The invention also relates to any stereoisomers, salts, solvates, and prodrugs of a compound of formula (I), including quaternary ammonium salts.

The compounds of the invention may contain one or more asymmetric center and may thus exist as different stereoisomeric forms. Accordingly, the present invention includes all possible stereoisomers and includes not only racemic compounds but the individual enantiomers and their non-racemic mixtures as well. When a compound is desired as a single enantiomer, such may be obtained by stereospecific synthesis, by resolution of the final product or any convenient intermediate, or by chiral chromatographic methods as each are known in the art. Resolution of the final product, an intermediate, or a starting material may be performed by any suitable method known in the art.

The compounds of the invention may be in the form of salts. Salts of the compounds of the invention include the acid addition and base salts thereof. Suitable acid addition salts are formed from acids. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen, phosphate/dihydrogen, phosphate, pyroglutamate, saccharate, stearate, succinate, tannate, tartrate, tosylate, trifluoroacetate and xinofoate salts. Suitable base salts are formed from bases. Examples include the aluminium, arginine, benzathine, calcium, choline, diethylamine, diolamine, glycine, lysine, magnesium, meglumine, olamine, potassium, sodium, tromethamine, 2-(diethylamino)ethanol, ethanolamine, morpholine, 4-(2-hydroxyethyl)morpholine and zinc salts. Hemisalts of acids and bases may also be formed, for example, hemisulphate and hemicalcium salts. When the compounds of the invention contain an acidic group as well as a basic group the compounds of the invention may also form internal salts, and such compounds are within the scope of the invention. When the compounds of the invention contain a hydrogen-donating heteroatom (e.g. NH), the invention also covers salts and/or isomers formed by transfer of said hydrogen atom to a basic group or atom within the molecule. Preferred salts include hydrochloride/chloride, hydrobromide/bromide, bisulphate/sulphate, nitrate, citrate, and acetate.

Salts of compounds of the invention may be prepared by one or more of these methods:
(i) by reacting the compound of the invention with the desired acid;
(ii) by reacting the compound of the invention with the desired base;
(iii) by removing an acid- or base-labile protecting group from a suitable precursor of the compound of the invention or by ring-opening a suitable cyclic precursor, for example, a lactone or lactam, using the desired acid; or
(iv) by converting one salt of the compound of the invention to another by reaction with an appropriate acid or by means of a suitable ion exchange column.

Optionally, one or more alkene, alkyne, oxo, hydroxyl, amido, amino, azido, nitro, borono, carboxylo, formyl, halo, haloformyl, phosphono, phosphato, thioxo or sulfhydryl being present in compound (I) is protected by any suitable protecting group known by a skilled person of the art.

According to an embodiment, R¹ and R² are each independently hydrogen, hydrocarbyl, aryl, heteroaryl, hydrocarbylaryl, arylhydrocarbyl, hydrocarbylheteroaryl, or heteroarylhydrocarbyl groups; and
the groups are optionally substituted by at least one group being hydrocarbyl, aryl, heteroaryl, oxo, hydroxyl, amido, amino, nitro, carboxylo, formyl, halo, thioxo or sulfhydryl; and
the groups are optionally interrupted or terminated by at least one group being -O-; -S-; or -NR^{N}- with R^{N} being hydrogen, hydrocarbyl, aryl, or a combination thereof; and
the nitrogen or sulfur atoms substituting or comprised in the groups are optionally oxidized.

According to a specific embodiment, R¹ and R² are each independently hydrogen, alkyl, alkenyl, aryl, heteroaryl, alkylaryl, arylalkyl, alkenylaryl, or arylalkenyl groups; and
the groups are optionally substituted by at least one group selected being hydroxyl, alkyl, alkenyl, aryl, alkylaryl, arylalkyl, amino, nitro, halo or sulfhydryl; and
the groups are optionally interrupted or terminated by at least one group being -O-; -S-; or -NR^{N}- with R^{N} being hydrogen, alkyl, alkenyl, aryl, or a combination thereof.

According to a more specific embodiment, R¹ and R² are each independently hydrogen, alkyl, alkenyl, aryl, heteroaryl, alkylaryl or alkenylaryl groups; and the groups are optionally substituted by at least one group being hydroxyl, alkyl, amino, nitro, halo or sulfhydryl; and the groups are optionally interrupted or terminated by at least one group being -O-; -S-; or -NR^{N}- with R^{N} being selected from the group consisting of hydrogen, alkyl, alkenyl, aryl, alkylaryl, or arylalkyl.

According to a furthermore specific embodiment, R¹ and R² are each independently hydrogen, alkyl, aryl or alkenylaryl groups; and the groups are optionally substituted by at least one halo group.

According to an embodiment, R¹ and R² form together a group being hydrocarbyl, aryl, heteroaryl, hydrocarbylaryl, arylhydrocarbyl, hydrocarbylheteroaryl, or heteroarylhydrocarbyl; and
the group is optionally substituted by at least one group being hydrocarbyl, heteroaryl, oxo, hydroxyl, amido, amino, nitro, carboxylo, formyl, halo, thioxo or sulfhydryl; and the group is optionally interrupted or terminated by at least one group being -O-; -S-; or -NR^{N}- with R^{N} being hydrogen, hydrocarbyl, aryl, or a combination thereof; and
the nitrogen or sulfur atoms substituting or comprised in the group are optionally oxidized.

According to a specific embodiment, R¹ and R² form together a group being alkyl, alkenyl, aryl, alkylaryl, arylalkyl, alkenylaryl or arylalkenyl;
the group is optionally substituted by at least one group being hydroxyl, alkyl, alkenyl, aryl, alkylaryl, arylalkyl, amino, nitro, halo or sulfhydryl; and
the group is optionally interrupted or terminated by at least one group being -O-; -S-; or -NR^{N}- with R^{N} being hydrogen, alkyl, alkenyl, aryl, or a combination thereof.

According to a more specific embodiment, R¹ and R² form together a group being alkyl, alkylaryl or arylalkyl; and the group is optionally substituted by at least one group being hydroxyl, alkyl, amino, nitro or halo; and the group is optionally interrupted or terminated by at least one group being -O- or -NR^{N}- with R^{N} being hydrogen, alkyl, alkenyl, aryl, alkylaryl, or arylalkyl.

According to a furthermore specific embodiment, R¹ and R² form together a group being alkyl or aryl.

According to an embodiment, R³ is hydrogen, an organic group or an organic molecule.

According to an embodiment, R³ is hydrogen, hydroxyl, amido, amino, cyano, tetrazolyl, triazolyl, nitro, carboxylo, formyl, halo or sulfhydryl.

According to an embodiment, R³ is any independent R¹ or R² group as previously disclosed.

According to an embodiment, R³ is a hydrogen, hydrocarbyl, aryl, heteroaryl, hydrocarbylaryl, arylhydrocarbyl, hydrocarbylheteroaryl, or heteroarylhydrocarbyl group;
the group is optionally substituted by at least one group being hydrocarbyl, heteroaryl, oxo, hydroxyl, amido, amino, azido, cyano, tetrazolyl, triazolyl, nitro, borono, carboxylo, formyl, halo, haloformyl, phosphono, phosphato, thioxo or sulfhydryl;
the group is optionally interrupted or terminated by at least one group being -B(OR^{B})- with R^{B} being hydrogen, hydrocarbyl, aryl, or a combination thereof; - O-; -PR^{P}- with R^{P} being hydrogen, hydrocarbyl, aryl, or a combination thereof; -P(OR^{OP})- with R^{OP} being hydrogen, hydrocarbyl, aryl, or a combination thereof; -S-; or -NR^{N}- with R^{N} being hydrogen, hydrocarbyl, aryl, or a combination thereof; or a combination thereof; and
the nitrogen, phosphorus or sulfur atoms substituting or comprised in the group are optionally oxidized.

According to an embodiment, R³ is a hydrogen, hydrocarbyl, aryl, heteroaryl, hydrocarbylaryl, arylhydrocarbyl, hydrocarbylheteroaryl, or heteroarylhydrocarbyl group;
the group is optionally substituted by at least one group being hydrocarbyl, aryl, heteroaryl, oxo, hydroxyl, amido, amino, tetrazolyl, triazolyl, nitro, carboxylo, formyl, halo, thioxo or sulfhydryl; and
the group is optionally interrupted or terminated by at least one group being -O-; -S-; or -NR^{N}- with R^{N} being hydrogen, hydrocarbyl, aryl, or a combination thereof; and
the nitrogen or sulfur atoms substituting or comprised in the group are optionally oxidized.

According to a specific embodiment, R³ is an alkyl, alkenyl, aryl, heteroaryl, alkylaryl, arylalkyl, alkenylaryl, arylalkenyl, alkylheteroaryl, or heteroarylalkyl group; and
the group is optionally substituted by at least one group being alkyl, alkenyl, aryl, heteroaryl, alkylaryl, arylalkyl, oxo, hydroxyl, amido, amino, tetrazolyl, triazolyl, nitro, carboxylo, formyl, halo, thioxo or sulfhydryl; and
the group is optionally interrupted or terminated by at least one group being -O-; -S-; or -NR^{N}- with R^{N} being hydrogen, alkyl, alkenyl, aryl, or a combination thereof.

According to a more specific embodiment, R³ is an alkyl, aryl, or alkylaryl group; and the group is optionally substituted by at least one group being oxo, hydroxyl, alkyl, amino or halo; and the group is optionally interrupted or terminated by at least one group being - O- or -NR^{N}- with R^{N} being hydrogen, alkyl, alkenyl, aryl, alkylaryl, or arylalkyl.

According to a furthermore specific embodiment, R³ is an alkyl or aryl group; and the group is optionally substituted by at least one group being oxo, hydroxyl, alkyloxyl, or halo.

According to a furthermore specific embodiment, the alpha-hydroxy-beta-triazole-tetrazolo is selected from the group consisting of:
(1-(4-phenyl-1H-1,2,3-triazol-1-yl)cyclohexyl)(1H-tetrazol-5-yl)methanol;
(1-(4-phenyl-1H-1,2,3-triazol-1-yl)cycloheptyl)(1H-tetrazol-5-yl)methanol;
(1-(4-phenyl-1H-1,2,3-triazol-1-yl)cyclooctyl)(1H-tetrazol-5-yl)methanol; tert-butyl 1-(1-(hydroxy(1H-tetrazol-5-yl)methyl)cyclooctyl)-1H-1,2,3-triazole-4-carboxylate;
(1-(4-(3-chloropropyl)-1H-1,2,3-triazol-1-yl)cyclooctyl)(1H-tetrazol-5-yl)methanol; and
2-(1-(1-(hydroxy(1H-tetrazol-5-yl)methyl)cyclooctyl)-1H-1,2,3-triazol-4-yl)ethan-1-ol.

According to another furthermore specific embodiment, the alpha-hydroxy-beta-triazolo-tetrazole is selected from the group consisting of:
2-ethyl-2-(4-phenyl-1H-1,2,3-triazol-1-yl)-1-(1H-tetrazol-5-yl)butan-1-ol;
2-(4-hexyl-1H-1,2,3-triazol-1-yl)-2-phenyl-1-(1H-tetrazol-5-yl)ethan-1-ol;
2-(4-hexyl-1H-1,2,3-triazol-1-yl)-2,2-diphenyl-1-(1H-tetrazol-5-yl)ethan-1-ol;
2-(4-(3-chloropropyl)- 1H-1,2,3-triazol-1-yl)-2,2-diphenyl-1-(1H-tetrazol-5-yl)ethan-1-ol; and
tert-butyl 1-(2-hydroxy-1,1-diphenyl-2-(1H-tetrazol-5-yl)ethyl)-1H-1,2,3-triazole-4-carboxylate.

According to an embodiment, R³ is a carbohydrate, an amino acid, a peptide or a nucleoside.

According to an embodiment, one or more amino, hydroxyl or sulfhydryl group being present in compound (I) is protected by any suitable protecting group known by a skilled person of the art.

According to a specific embodiment, one or more amino group being present in compound (I) is protected by a protective group being benzyl (CH₂Ph), *p*-methoxybenzyl ether (PMB), *tert*-butyloxycarbonyl (BOC), carbobenzoyloxy (Cbz) or tosyl (Ts).

According to another specific embodiment, one or more hydroxyl group being present in compound (I) is protected by a protective group being benzyl (CH₂Ph), *p*-methoxybenzyl ether (PMB), tetrahydropyranyl (THP) or silyl ether such as trimethylsilyl (TMS, SiMe₃), *tert-*butyldimethylsilyl (TBDMS, Si*t*Bu(Me)₂), triethylsilyl (TES, SiEt₃), methyldiphenylsilyl (SiPh₂Me) or tri-isopropylsilyl (TIPS, Si(iPr)₃).

According to another specific embodiment, one or more sulfhydryl group being present in compound (I) is protected by a protective group being benzyl (CH₂Ph), *p*-methoxybenzyl ether (PMB), triphenylmethyl ((C₆H₅)₃C) or tetrahydropyranyl (THP).

### Synthesis of alpha-hydroxy-beta-triazolo-tetrazoles - CuAAC reaction

In its second aspect, the invention relates to a process for manufacturing a alpha-hydroxy-beta-triazolo-tetrazole, comprising carrying out the reaction between an alpha-hydroxy-beta-azido-tetrazole and a terminal alkyne in presence of a copper(I) source and a tertiary amine.

Alpha-hydroxy-beta-azido-tetrazoles may be synthesized by the method recently published by Quinodoz et al. (Quinodoz, P. et al., Organic Chemistry Frontiers, 2015, Vol. 2, pp. 492-496.).

According to an embodiment, the invention relates to a process for manufacturing an alpha-hydroxy-beta-triazolo-tetrazole of formula (I): 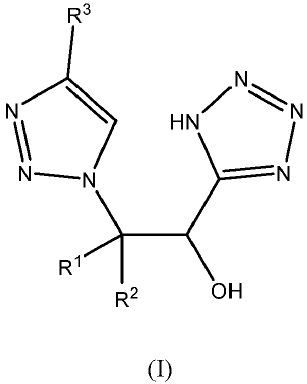 comprising starting from an alpha-hydroxy-beta-azido-tetrazole of formula (II): 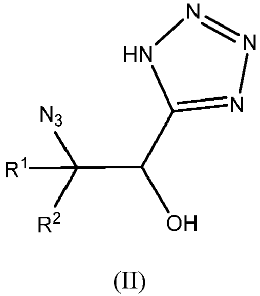 wherein R¹ and R² are each independently groups as previously disclosed; or R¹ and R² form together a group as previously disclosed;
and a terminal alkyne of formula R³-C≡C-H,
wherein R³ is as previously disclosed; and carrying out the reaction of compound (II) with alkyne R³-C≡C-H in presence of a copper(I) source and a tertiary amine, wherein the copper(I) source is either:
- a combination of a copper(I) salt and a base, or
- a combination of a copper(II) salt and a reducing agent.

This process is schematically represented below (L referring to the tertiary amine):

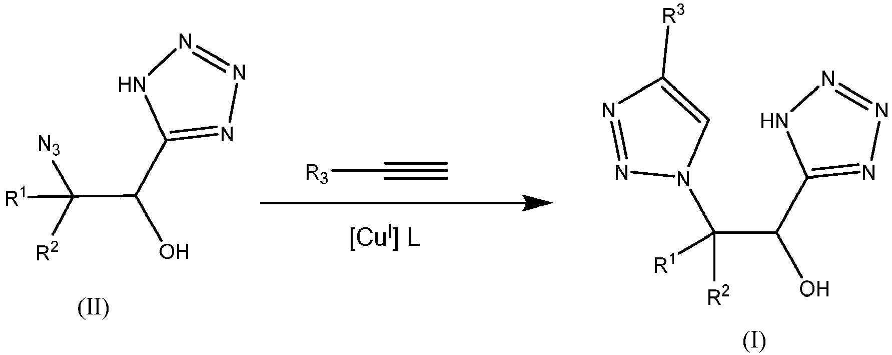

According to an embodiment, the copper(I) salt is copper(I) chloride, copper(I) bromide (CuBr) or copper(I) acetate; and the base is a basic amine such as N,N-diisopropylethylamine (DIEA). According to a specific embodiment, the copper salt is copper(I) bromide and the base is N,N-diisopropylethylamine.

According to another embodiment, the copper(II) salt is copper(II) chloride, copper(II) bromide, copper(II) acetate or copper(II) sulphate (Cu^{II}SO₄); and the reducing agent is sodium ascorbate (NaAs) or tri(2-carboxyethyl)phosphine (TECP). According to a specific embodiment, the copper salt is copper(II) sulphate and the reducing agent is sodium ascorbate.

According to an embodiment, the tertiary amine is tris(benzyltriazolylmethyl)amine (TBTA), tris(*tert*butyltriazolylmethyl)amine (TTTA), *tris*(benzimidazole)methyl amine (TBIA), 4,7-diphenyl-1,10-phenanthroline-disulfonic acid disodium salt, tris[2-(N,N-dibenzylamino)ethyl]amine or tris(benzyltriazolylmethyl)amine. According to a specific embodiment, the tertiary amine is tris(benzyltriazolylmethyl)amine (TBTA).

According to an embodiment, the alkyne/azide molar ratio in the reaction medium ranges from 1 to 10. According to a specific embodiment, the alkyne/azide ratio ranges from 2 to 5. According to a more specific embodiment, the alkyne/azide ratio is about 3 (i.e. about 3 equiv. of alkyne for 1 equiv. of azide).

According to an embodiment, the copper salt/azide molar ratio in the reaction medium ranges from 0.01 to 2. According to a specific embodiment, the copper salt/azide ratio ranges from 0.05 to 0.2. According to a more specific embodiment, the copper salt/azide ratio is about 0.1 (i.e. about 0.1 equiv. of copper salt for 1 equiv. of azide).

According to an embodiment, the copper salt/tertiary amine molar ratio in the reaction medium ranges from 0.1 to 10. According to a specific embodiment, the copper salt/tertiary amine ratio ranges from 0.5 to 2. According to a more specific embodiment, the copper salt/tertiary amine ratio is about 1 (i.e. about 1 equiv. of copper salt for 1 equiv. of tertiary amine).

According to an embodiment, the copper salt/reducing agent molar ratio in the reaction medium ranges from 0.1 to 2. According to a specific embodiment, the copper salt/ reducing agent ratio ranges from 0.25 to 0.75. According to a more specific embodiment, the copper salt/reducing agent ratio is about 0.5 (i.e. about 0.5 equiv. of copper salt for 1 equiv. of reducing agent).

According to an embodiment, the step of carrying out the reaction between the alpha-hydroxy-beta-azido-tetrazole and the terminal alkyne is executed in a solvent. According to a specific embodiment, the solvent is ethanol, tetrahydrofurane (THF), N,N'-dimethylformamide (DMF), acetonitrile, *n*-butyl alcohol (*n*-BuOH), water or mixtures thereof. According to a more specific embodiment, the solvent is a mixture of *n*-butyl alcohol and water.

According to an embodiment, the step of carrying out the reaction between the alpha-hydroxy-beta-azido-tetrazole and the terminal alkyne is executed in a duration ranging from 12h to 5 days. According to a specific embodiment, the duration ranges from 24h to 72h. According a more specific embodiment, the duration is about 48h.

According to an embodiment, the step of carrying out the reaction between the alpha-hydroxy-beta-azido-tetrazole and the terminal alkyne is executed at a temperature ranging from 0 to 50°C. According to a specific embodiment, the temperature ranges from 15 to 30°C. According to a more specific embodiment, the temperature is room temperature, i.e. about 25°C.

According to an embodiment, the step of carrying out the reaction between the alpha-hydroxy-beta-azido-tetrazole and the terminal alkyne is executed under a suitable inert gas, such as argon.

According to a specific embodiment, the invention relates to a process for manufacturing a alpha-hydroxy-beta-triazolo-tetrazole of formula (III) as previously disclosed comprising starting from an alpha-hydroxy-beta-azido-tetrazole of formula (I) and a terminal alkyne of formula R³-C≡C-H as previously disclosed, and carrying out the reaction of compound (II) with alkyne R³-C≡C-H in presence of Cu^{II}SO₄, TBTA and NaAs in a *n*-BuOH/H₂O mixture.

During the reaction described in this aspect of the invention, the hydroxy-tetrazole group acts as a "latent" or "hidden" alkyne group. This means that it may be easily converted to an alkyne (as described hereafter) but does not react under CuAAC conditions. Such properties are highly desirable in order to achieve controlled orthogonal CuAAC reactions.

### Triazole alkynes

Wardrop et al. described the conversion of alpha-hydroxy-tetrazoles to alkynes through a [1,2]-rearrangement process using carbodiimides, which are compounds of general formula RN=C=NR, as dehydrating agents (Wardrop, D. J. et al., Organic Letters, 2012, Vol. 14, No. 6, pp. 1548-1551.). From alpha-hydroxy-alpha-aryl tetrazoles, this reaction allowed the preparation of internal alkynes substituted by at least one aryl group, most conveniently using diisoprolylcarbodiimide (DIC). However, instead of forming this alkyne, this process could also lead to a 5-membered cycle or heterocycle, by a cyclisation mechanism involving an [1,5]-C - H insertion.

In its third aspect, the invention relates to a triazole alkyne of formula (III): 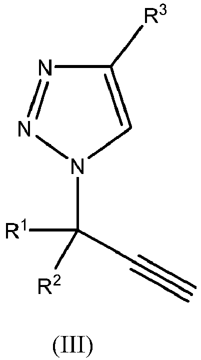 wherein R¹ and R² are each independently groups as previously disclosed; or R¹ and R² form together a group as previously disclosed; and R³ is as previously disclosed.

The invention also relates to any stereoisomers, salts, solvates, and prodrugs of a compound of formula (III), including quaternary ammonium salts.

According to a furthermore specific embodiment, the triazole alkynes manufactured by the process are selected from the group consisting of:
1-(1-ethynylcyclohexyl)-4-phenyl-1H-1,2,3-triazole;
1-(1-ethynylcycloheptyl)-4-phenyl-1H-1,2,3-triazole;
1-(1-ethynylcyclooctyl)-4-phenyl-1H-1,2,3-triazole;
tert-butyl 1-(1-ethynylcyclooctyl)-1H-1,2,3-triazole-4-carboxylate;
4-(3-chloropropyl)-1-(1-ethynylcyclooctyl)-1H-1,2,3-triazole;
2-(1-(1-ethynylcyclooctyl)-1H-1,2,3-triazol-4-yl)ethan-1-ol;
1-(3-ethylpent-1-yn-3-yl)-4-phenyl-1H-1,2,3-triazole;
4-hexyl-1-(1-phenylprop-2-yn-1-yl)-1H-1,2,3-triazole; and
1-(1,1-diphenylprop-2-yn-1-yl)-4-hexyl-1H-1,2,3-triazole.

### Synthesis of triazole alkynes - Hydroxy-tetrazole to alkyne reaction

In its fourth aspect, the invention relates to a process for manufacturing a triazole alkyne, comprising carrying out the reaction between an alpha-hydroxy-beta-triazolo-tetrazole and a carbodiimide.

According to an embodiment, the invention relates to a process for manufacturing a triazole alkyne of formula (III): 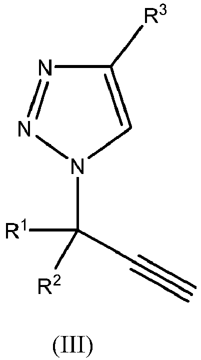 comprising starting from an alpha-hydroxy-beta-triazolo-tetrazole of formula (I): 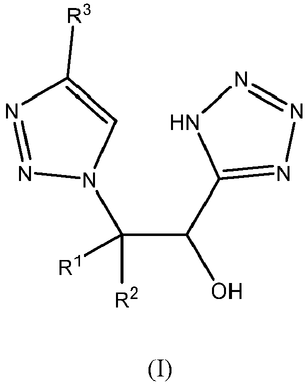 wherein R¹ and R² are each independently groups as previously disclosed; or R¹ and R² form together a group as previously disclosed; and R³ is a group as previously disclosed; and carrying out the reaction of compound (I) with a carbodiimide.

This process is schematically represented below:

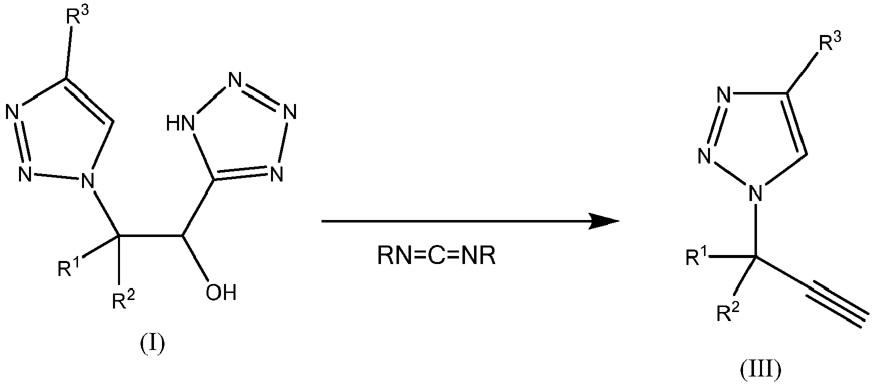

According to an embodiment, the carbodiimide is N,N'-dicyclohexylcarbodiimide (DCC), N, N'-diisopropylcarbodiimide (DIC), 1-ethyl-3-(3-dimethylaminopropyl)carbodiimide (EDC), phenyl ethyl carbodiimide (PEC), phenyl isopropyl carbodiimide (PIC), *tert*-butyl ethyl carbodiimide (BEC) or *tert*-butyl methyl carbodiimide (BMC). According to a specific embodiment, the carbodiimide is EDC.

According to an embodiment, the carbodiimide/tetrazole molar ratio in the reaction medium ranges from 0.5 to 5. According to a specific embodiment, the carbodiimide/tetrazole ratio ranges from 1 to 1.5. According to a more specific embodiment, the carbodiimide/tetrazole ratio is about 1.2 (i.e. about 1.2 equiv. of carbodiimide for 1 equiv. of tetrazole).

According to an embodiment, the step of carrying out the reaction between the alpha-hydroxy-beta-triazolo-tetrazole and the carbodiimide is executed in a solvent. According to a specific embodiment, the solvent is: chloroform, 1,2-dichloroethane, dichloromethane or mixture thereof. According to a more specific embodiment, the solvent is dichloromethane.

According to an embodiment, the step of carrying out the reaction between the alpha-hydroxy-beta-triazolo-tetrazole and the carbodiimide is executed in a duration ranging from 4h to 72h. According to a specific embodiment, the duration ranges from 12h to 24h. According a more specific embodiment, the duration is about 18h.

According to an embodiment, the step of carrying out the reaction between the alpha-hydroxy-beta-triazolo-tetrazole and the carbodiimide is executed at a temperature ranging from 0 to 50°C. According to a specific embodiment, the temperature ranges from 15 to 30°C. According to a more specific embodiment, the temperature is room temperature, i.e. about 25°C.

According to a specific embodiment, the invention relates to a process for manufacturing an triazole alkyne of formula (IV) as previously disclosed, comprising starting from an alpha-hydroxy-beta-triazolo-tetrazole of formula (I) as previously disclosed, and carrying out the reaction of compound (I) with EDC in dichloromethane.

The reaction described in this aspect of the invention allows the conversion of the hydroxy-tetrazole to an alkyne, revealing the "latent" or "hidden" alkyne group of the alpha-hydroxy-beta-triazolo-tetrazole.

### Multi-triazoles - Further CuAAC reactions

In its fifth aspect, the invention relates to a process for manufacturing molecules comprising starting from a triazole alkyne of formula (III), and carrying out the reaction of the compound (III) with an azide.

According to a first embodiment, the invention relates to a process for manufacturing a di-triazole of formula (IV): 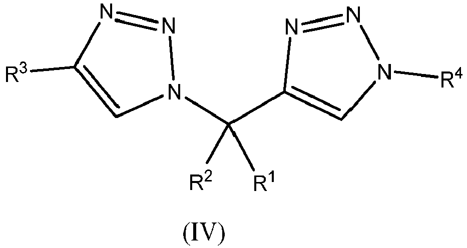 comprising starting from a triazole alkyne of formula (III): 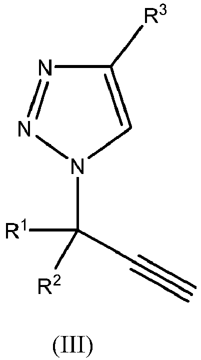 wherein R¹ and R² are each independently groups as previously disclosed; or R¹ and R² form together a group as previously disclosed; and R³ is as previously disclosed;
and from an azide of formula R⁴-N₃,
wherein R⁴ is any R³ group as previously disclosed; and carrying out the reaction of compound (IV) with azide R⁴-N₃ in presence of a copper(I) source.

This process is schematically represented below:

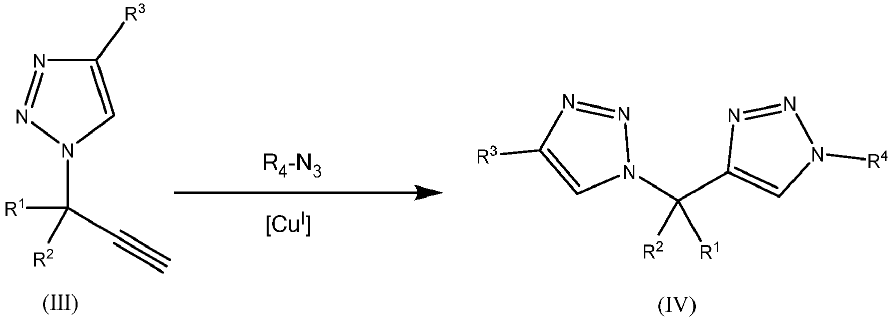

In this first embodiment, the compounds and the conditions of the step of reaction of compound (III) with azide R⁴-N₃ may be as previously disclosed in any embodiment of previous paragraph entitled "*synthesis of alpha*-*hydroxy*-*beta*-*triazole*-*tetrazoles* - *CuAAC reaction*", except that the presence of a tertiary amine ligand may not be required.

According to a specific embodiment, R⁴ is a carbohydrate.

According to a specific embodiment, R⁴ is a metal complex, such as an iron complex.

According to a specific embodiment, R⁴ is an alkyl or alkylaryl group; optionally substituted by at least one group being alkyl, oxo, hydroxyl, tetrazolyl or halo; and optionally interrupted or terminated by at least one group being -O- or -NH-.

According to a more specific embodiment, the di-triazoles manufactured by this process are selected from the group consisting of:
1-octyl-4-(1-(4-phenyl-1H-1,2,3-triazol-1-yl)cyclohexyl)-1H-1,2,3-triazole;
1-[bis(η5-cyclopentadienyl)iron]-4-(1-(4-phenyl-1H-1,2,3-triazol-1-yl)cyclohexyl)-1H-1,2,3-triazole;
2-(acetoxymethyl)-6-(4-(1-(4-phenyl-1H-1,2,3-triazol-1-yl)cyclohexyl)-1H-1,2,3-triazol-1-yl)tetrahydro-2H-pyran-3,4,5-triyl triacetate;
methyl 2-((tert-butoxycarbonyl)amino)-3-(4-(1-(4-phenyl-1H-1,2,3-triazol-1-yl)cyclohexyl)-1H-1,2,3-triazol-1-yl)propanoate;
1-octyl-4-(1-(4-phenyl-1H-1,2,3-triazol-1-yl)cyclooctyl)-1H-1,2,3-triazole;
ethyl 4-(4-(1-(4-phenyl-1H-1,2,3-triazol-1-yl)cyclooctyl)-1H-1,2,3-triazol-1-yl)butanoate;
4-phenyl-1-(1-(1-(pyren-1-ylmethyl)-1H-1,2,3-triazol-4-yl)cyclooctyl)-1H-1,2,3-triazole;
1-benzyl-4-(1-(4-phenyl-1H-1,2,3-triazol-1-yl)cyclooctyl)-1H-1,2,3-triazole;
2-ethyl-2-(4-(1-(4-phenyl-1H-1,2,3-triazol-1-yl)cyclooctyl)-1H-1,2,3-triazol-1-yl)-1-(1H-tetrazol-5-yl)butan-1-ol.

According to a second embodiment, the invention relates to a process for manufacturing an alpha-hydroxy-tetrazole of formula (V): 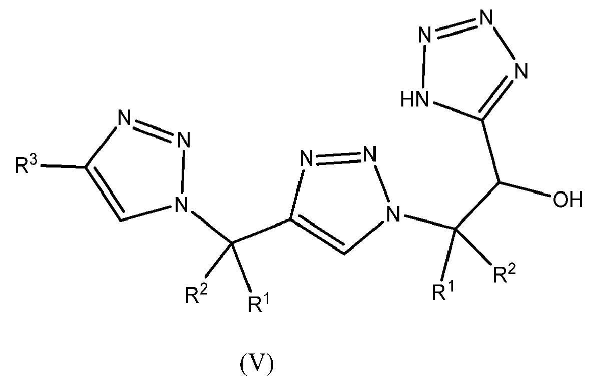 comprising starting from a triazole alkyne of formula (III): 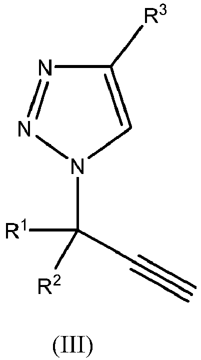 and from an alpha-hydroxy-beta-azido tetrazole of formula (II): 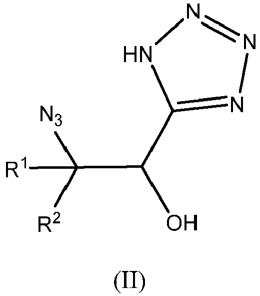 wherein R¹ and R² are each independently groups as previously disclosed; or R¹ and R² form together a group as previously disclosed; and R³ is as previously disclosed;
and carrying out the reaction of the compound (III) with compound (II) in presence of a copper(I) source and a tertiary amine.

According to a third embodiment, the invention relates to a process for manufacturing a poly-triazole compound of formula (VI): 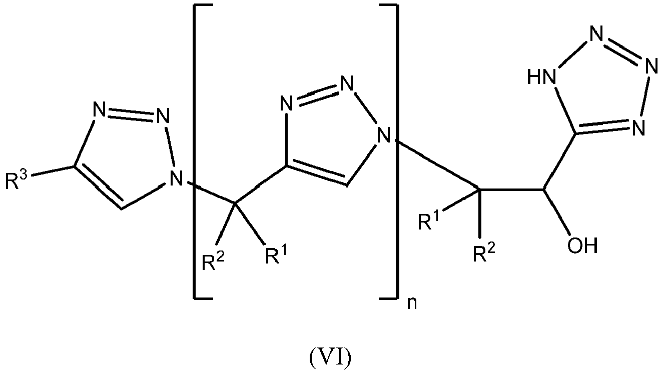 comprising starting from a triazole alkyne of formula (III): 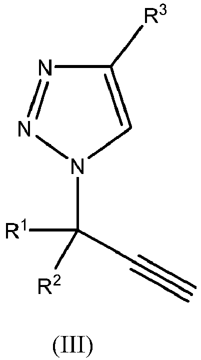 and from an alpha-hydroxy-beta-azido tetrazole of formula (II): 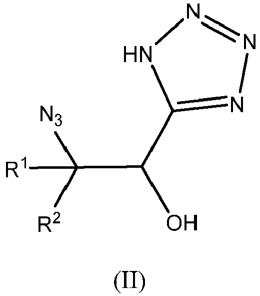 wherein R¹ and R² are each independently groups as previously disclosed; or R¹ and R² form together a group as previously disclosed; and R³ is as previously disclosed;
and performing n iterations of the following steps (a) and (b):
(a) carrying out the reaction of the compound (III) with compound (II) in presence of a copper(I) source and a tertiary amine.
(b) carrying out the reaction of the resulting compound with a carbodiimide.

This process is schematically represented below (L referring to the tertiary amine):

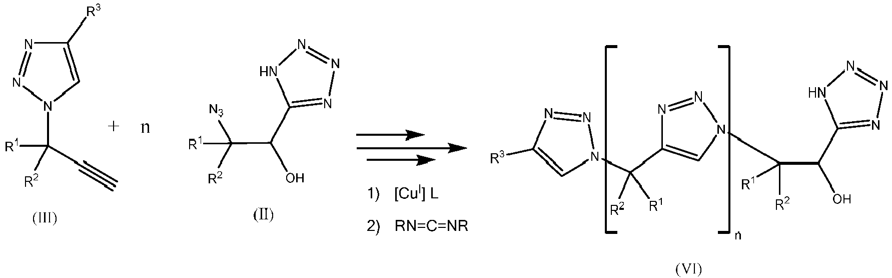

According to a specific embodiment, n ranges from 0 to 100. According to a more specific embodiment, n ranges from 1 to 10. According to another more specific embodiment, n ranges from 2 to 5.

According to a more specific embodiment, the poly-triazoles manufactured by this process are selected from the group consisting of:
1-(3-ethylpent-1-yn-3-yl)-4-(1-(4-phenyl-1H-1,2,3-triazol-1-yl)cyclooclyl)-1H-1,2,3-triazole;
1-(1-ethynylcyclooctyl)-4-(3-(4-(1-(4-phenyl-1H-1,2,3-triazol-1-yl)cyclooclyl)-1H-1,2,3-triazol-1-yl)pentan-3-yl)-1H-1,2,3-triazole; and
1-benzyl-4-(1-(4-(3-(4-(1-(4-phenyl-1H-1,2,3-triazol-1-yl)cyclooclyl)-1H-1,2,3-triazol-1-yl)pentan-3-yl)-1H-1,2,3-triazol-1-yl)cyclooctyl)-1H-1,2,3-triazole.

In these second and third embodiments, the compounds and the conditions of step (a) may be as previously disclosed in any embodiment of previous paragraph entitled *"synthesis of alpha*-*hydroxy*-*beta*-*triazolo*-*tetrazoles* - *CuAAC reaction*".

In this third embodiment, the compounds and the conditions of steps (b) may be as previously disclosed in any embodiment of previous paragraph entitled "*Triazole alkynes - Hydroxy*-*tetrazole to alkine reaction*".

The triazole heterocycle is an analogue of the peptidic bound, therefore oligomeric or polymeric compounds of general formula (VI) are potential peptides analogues. Such biomimetic compounds may present valuable applications in medicinal chemistry, and more generally in the biotechnologies field.

### EXAMPLES

The present invention is further illustrated by the following examples.

### General materials and methods

### Materials

Azides were prepared using known methods of the literature: Octyl azide (Org. Biomol. Chem., 2012, 10, 5993-6002.), Ferrocenyl azide (J. Organometal. Chem., 1970, 23, 225-228.), Pyrene azide (J. Org. Chem. 2008, 73, 8212-8218.), Methyl 3-azido-2-(*tert*butoxycarbonyl-amino)propanoate *(*Bioorg. Med. Chem., 2010, 18, 7338-7347.), 2,3,4,6-Tetra-O-acetyl-1-azido-β-D-glucopyranoside (Tet. Lett. 2007 48 3953-3957.), Ethyl 4-azido-butyrate (Eur. J. Org. Chem., 2011, 229-233.). Benzyl azide was purchased from Sigma-Aldrich.

Alkynes, other reactants and solvent are commercially available from ordinary chemical compounds suppliers, and were purchased from Sigma-Aldrich, Alfa Aesar, Acros Organics or TCI Chemicals.

### Methods

Column chromatography were performed on a silica gel 230-400 mesh by using various mixtures of dichloromethane (DCM), ethyl acetate (EtOAc), methanol (MeOH), acetic acid (AcOH) and petroleum ether (PE). Thin Layer chromatographies (TLCs) were run on Kieselgel 60F₂₅₄ plates and revealed by UV light and potassium permanganate or ninhydrin.

¹H and ¹³C NMR spectra were collected on a Bruker Avance spectrometer respectively at 200 or 300 MHz and 75 MHz. Data are presented as follows: chemical shift (in ppm on the δ scale relative to δTMS = 0), multiplicity (s = singlet, d = doublet, t = triplet, m = multiplet, b = broad), coupling constant (J/Hz), integration and attribution. High resolution mass spectra (HR-MS) were obtained on a Waters Micromass Q-TofMicro instrument. Melting points are uncorrected.

### Example 1: Alpha-hydroxy-beta-triazolo-tetrazoles

Hereafter are provided alpha-hydroxy-beta-triazole-tetrazoles according to the invention. R¹, R² and R³ groups refer to formula (I):

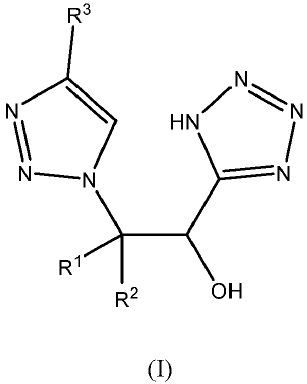

| **#** | **R¹** | **R²** | **R³** | **Formula** | **Name** |
|---|---|---|---|---|---|
| **1** | pentyl (cyclo hexyl) | | Ph | 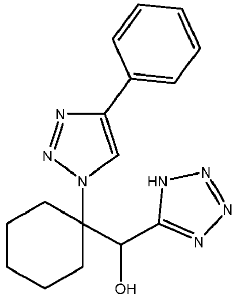 | (1-(4-phenyl-1H-1,2,3-triazol-1-yl)cyclohexyl)(1H-tetrazol-5-yl)methanol |
| **2** | hexyl (cyclo heptyl) | | Ph | 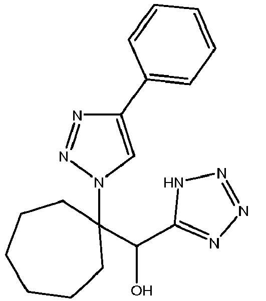 | (1-(4-phenyl-1H-1,2,3-triazol-1-yl)cycloheptyl)(1H -tetrazol-5-yl)methanol |
| **3** | heptyl (cyclo octyl) | | Ph | 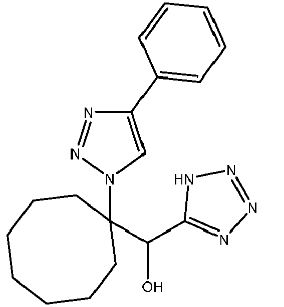 | (1-(4-phenyl-1H-1,2,3-triazol-1-yl)cyclooctyl)(1H-tetrazol-5-yl)methanol |
| **4** | heptyl (cyclo octyl) | | -COO*t*Bu | 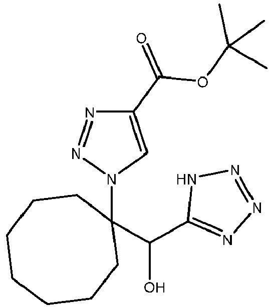 | tert-butyl1-(1-(hydroxy(1H-tetrazol-5-yl)methyl)cyclooct yl)-1H-1,2,3-triazole-4-carboxylate |
| **5** | heptyl (cyclo octyl) | | 3-chloropropyl | 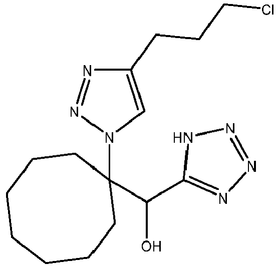 | (1-(4-(3-chloropropyl)-1H-1,2,3-triazol-1-yl)cyclooctyl)(1H-tetrazol-5-yl)methanol |
| **6** | heptyl (cyclo octyl) | | 2-hydroxyethyl | 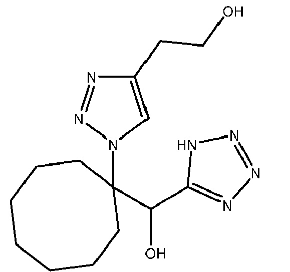 | 2-(1-(1-(hydroxy(1H-tetrazol-5-yl)methyl)cyclooct yl)-1H-1,2,3-triazol-4-yl)ethan-1-ol |
| **7** | Et | Et | Ph | 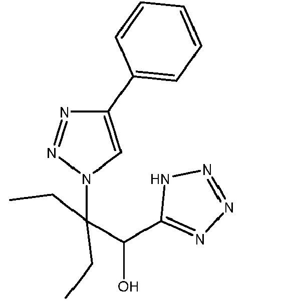 | 2-ethyl-2-(4-phenyl-1H-1,2,3-triazol-1-yl)-1-(1H-tetrazol-5-yl)butan-1-ol |
| **8** | Ph | H | hexyl | 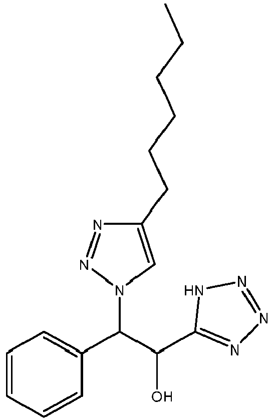 | 2-(4-hexyl-1H-1,2,3-triazol-1-yl)-2-phenyl-1-(1H-tetrazol-5-yl)ethan-1-ol |
| **9** | Ph | Ph | hexyl | 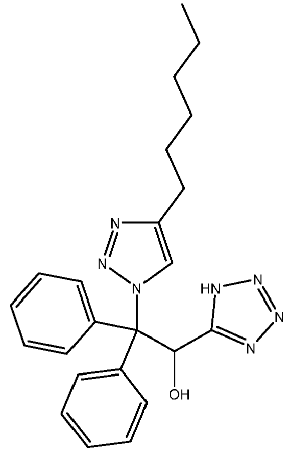 | 2-(4-hexyl-1H-1,2,3-triazol-1-yl)-2,2-diphenyl-1-(1H-tetrazol-5-yl)ethan-1-ol |
| **10** | Ph | Ph | 3-chloro propyl | 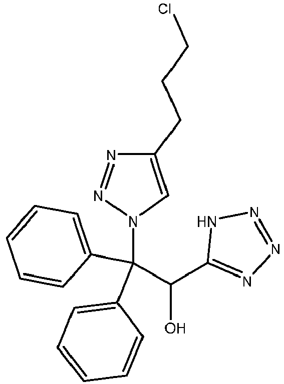 | 2-(4-(3-chloropropyl)-1H-1,2,3-triazol-1-yl)-2,2-diphenyl-1-(1H-tetrazol-5-yl)ethan-1-ol |
| **11** | Ph | Ph | -COO*t*Bu | 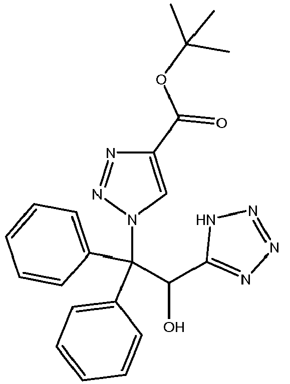 | tert-butyl 1-(2-hydroxy-1,1-diphenyl-2-(1H-tetrazol-5-yl)ethyl)-1H-1,2,3-triazole-4-carboxylate |

These compounds may be prepared as disclosed in Example 2.

### Example 2: CuAAC synthesis of alpha-hydroxy-beta-triazolo-tetrazoles

### Materials and Methods

Procedure for the synthesis of alpha-hydroxy-beta-azido-tetrazoles: these starting compounds for the synthesis of alpha-hydroxy-beta-triazolo-tetrazoles were prepared from the appropriate epoxynitriles, following the procedure disclosed in Quinodoz, P. et al., Organic Chemistry Frontiers, 2015, Vol. Vol. 2, pp. 492-496. and its Supplementary Material.

### Procedures for the synthesis of alpha-hydroxy-beta-tetrazolo-tetrazoles

Procedure (a): The alpha-hydroxy-beta-azido-tetrazole substrate (1 mmol) was dissolved in *n*-BuOH (3 mL). An alkyne (3 mmol) and TBTA (tris((1-benzyl-1H-1,2,3-triazolyl)methyl)amine) (0.1 mmol) were added. A solution of sodium ascorbate (0.2 mmol in 1.5 mL water) was added, followed by a solution of copper sulphate (0.1 mmol in 1.5 mL water). The mixture was stirred at room temperature for 48 hours. The organic phase was separated and concentrated under reduced pressure. The residue was purified by flash chromatography on silica gel.

Procedure (b): A mixture of alpha-hydroxy-beta-azido-tetrazole substrate (0.10 mmol), copper (I) bromide (0.01 mmol) and TBTA (0.011 mmol) in THF (1 mL) was placed under argon atmosphere. An alkyne (0.30 mmol) and diisopropylethylamine (0.05 mL, 0.30 mmol) were added, and the mixture was stirred for 24 hours. The mixture was concentrated under reduced pressure. The residue was purified by flash chromatography on silica gel.

### Results

Hereafter are provided the yield and physical characterization of alpha-hydroxy-beta-triazolo-tetrazoles according to the invention, prepared by procedure (a). Compound #3 was also prepared by procedure (b).

Compounds #1-11 were prepared from the corresponding alpha-hydroxy-beta-azido-tetrazoles and from the appropriate R³-C≡CH terminal alkyne.

| **#** | **Name** | **Characterization** | **Yield** |
|---|---|---|---|
| **1** | (1-(4-phenyl-1H-1,2,3-triazol-1-yl)cyclohexyl)( 1H -tetrazol-5-yl)methanol | Mp: 210-212°C.¹H NMR (300 MHz, DMSO-d6) δ 8.60 (s, 1H), 7.87 (m, 2H), 7.44 (m, 2H), 7.31 (m, 1H), 6.76 (d, *J* = 5.0 Hz, 1H), 5.15 (d, *J* = 5.0 Hz, 1H), 2.61-2.66 (m, 2H), 1.88-1.96 (m, 2H), 1.48-1.67 (m, 3H), 1.08-1.29 (m, 3H). ¹³C NMR (75 MHz, DMSO-d6) δ 155.9, 145.6, 131.1, 128.7, 127.6, 125.1, 121.5, 70.9, 66.3, 30.3, 24.3, 20.9, 20.8. HRMS (ESI, TOF MS) m/z calculated for C₁₆H₂₀N₇O [M+H]⁺: 326.1729, found: 326.1719. | 84% (a) |
| **2** | (1-(4-phenyl-1H-1,2,3-triazol-1-yl)cycloheptyl)1 H-tetrazol-5-yl)methanol | Mp: 119-120°C.¹H NMR (300 MHz, DMF-d6) δ 8.74 (s, 1H), 7.98 (m, 2H), 7.47 (m, 2H), 7.35 (m, 1H), 7.03 (bs, 1H), 5.46 (bs, 1H), 3.53 (bs, 1H), 2.32-2.36 (m, 2H), 1.88-1.96 (m, 2H), 1.45-1.54 (m, 8H). ¹³C NMR (75 MHz, DMF-d6) δ 147.3, 132.8, 130.0, 129.8, 128.6, 126.3, 122.6, 72.8, 71.9, 30.4, 23.2, 23.1, 21.7. HRMS (ESI, TOF MS) m/z calculated for C₁₇H₂₂N₇O [M+H]⁺: 340.1886, found: 340.1893. | 98% (a) |
| **3** | (1-(4-phenyl-1H-1,2,3-triazol-1-yl)cyclooctyl)( 1H -tetrazol-5-yl)methanol | Mp: 86-88°C. ¹H NMR (300 MHz, DMF-d6) δ 8.71 (s, 1H), 7.96 (m, 2H), 7.44-7.49 (m, 2H), 7.34 (m,, 1H), 7.03 (bs, 1H), 5.48 (s, 1H), 3.66 (bs, 1H), 2.52-2.72 (m, 3H), 2.36-2.43 (m, 1H), 1.47-1.77 (m, 12H). ¹³C NMR (75 MHz, DMF-d6) δ 157.7, 146.9, 132.8, 129.8, 128.6, 126.3, 122.7, 80.2, 71.7, 71.6, 29.3, 28.5, 25.7, 22.8. HRMS (ESI, TOF MS) m/z calculated for C₁₅H₂₄N₇O [M+H]⁺: 354.2042, found: 354.2045. | 98% (a) 90% (b) |
| **4** | tert-butyl 1-(1-(hydroxy(1H-tetrazol-5-yl)methyl)cyclooc tyl)-1H-1,2,3-triazole-4-carboxylate | Mp: 118-120°C.¹H NMR (300 MHz, DMSO-d6) δ 8.60 (s, 1H), 6.83 (bs, 1H), 5.25 (s, 1H), 2.25-2.45 (m, 3H), 1.26-1.66 (m, 20H). ¹³C NMR (75 MHz, DMSO-d6) δ 159.9, 139.5, 128.9, 81.1, 69.8, 29.1, 27.9, 26.3, 24.2, 21.3. HRMS (ESI, TOF MS) m/z calculated for C₁₇H₁₈N₇O₃ [M+H]⁺: 378.2254, found: 378.2257. | 98% (a) |
| **5** | (1-(4-(3-chloropropyl)-1H-1,2,3-triazol-1-yl)cyclooctyl)(1H -tetrazol-5-yl)methanol | Mp: 85-87°C. ¹H NMR (300 MHz, CDCl₃) δ 7.23 (s, 1H), 5.51 (s, 1H), 3.45 (t, *J* = 5.9 Hz, 1H), 2.67-2.79 (m, 4H), 2.42-2.51 (m, 2H), 2.01-2.04 (m, 2H), 1.47-1.82 (m, 10H). ¹³C NMR (75 MHz, CDCl₃) δ 121.8, 72.1, 71.3, 43.7, 31.6, 30.4, 29.6, 28.2, 27.3, 24.9, 22.1, 22.0. HRMS (ESI, TOF MS) m/z calculated for C₁₅H₂₅N₇OCl [M+H]⁺: 354.1809, found: 354.1810. | 71% (a) |
| **6** | 2-(1-(1-(hydroxy(1H-tetrazol-5-yl)methyl)cyclooc tyl)-1H-1,2,3-triazol-4-yl)ethan-1-ol | ¹H NMR (300 MHz, CD₃OD) δ 7.87 (s, 1H), 5.28 (s, 1H), 3.83 (t, *J* = 6.7 Hz, 1H), 2.90 (t, *J* = 6.5 Hz, 1H), 2.30-2.61 (m, 4H), 1.43-1.57 (m, 10H). ¹³C NMR (75 MHz, CDCl₃) δ 158.7, 145.3, 124.4, 72.2, 72.0, 62.2, 30.9, 30.2, 30.0, 29.6, 28.8, 26.0, 23.1. HRMS (ESI, TOF MS) m/z calculated for C₁₄H₂₄N₇O₂ [M+H]⁺: 322.1991, found: 322.1995. | 94% (a) |
| **7** | 2-ethyl-2-(4-phenyl-1H-1,2,3-triazol-1-yl)-1-(1H-tetrazol-5-yl)butan-1-ol | Mp: 103-105°C.¹H NMR (300 MHz, DMSO-d6) δ 8.56 (s, 1H), 7.87 (m, 2H), 7.43 (m,, 2H), 7.30 (m, 1H), 6.84 (d, *J* = 5.4 Hz, 1H), 5.34 (d, *J* = 5.4 Hz, 1H), 2.18-2.36 (m, 4H), 0.90 (t, *J* = 7.3 Hz, 3H), 0.83 (t, *J* = 7.4 Hz, 3H).¹³C NMR (75 MHz, DMSO-d6) δ 156.2, 145.3, 131.1, 128.7, 127.6, 125.1, 121.5, 69.9, 68.3, 24.6, 23.8, 7.5. HRMS (ESI, TOF MS) m/z calculated for C₁₅H₂₀N₇O [M+H]⁺: 314.1729, found: 314.1724. | 99% (a) |
| **8** | 2-(4-hexyl-1H-1,2,3-triazol-1-yl)-2-phenyl-1-(1H-tetrazol-5-yl)ethan-1-ol | Mp: 157-159°C. ¹H NMR (300 MHz, DMF-d6) δ 8.32 (bs, 1H), 7.30-7.53 (m, 5H), 6.37 (b, 2H), 2.66 (t, *J* = 7.3 Hz, 1H), 1.61-1.64 (m, 2H), 1.30 (b, 6H), 0.87 (t, *J* = 5.8 Hz, 1H). ¹³C NMR (75 MHz, DMF-d6) δ 136.7, 128.8, 128.3, 122.2, 68.6, 66.9, 31.7, 28.9, 25.7, 22.6, 13.8. HRMS (ESI, TOF MS) m/z calculated for C₁₇H₁₄N₇O [M+H]⁺: 342.2042, found: 342.2049. | 78% (a) |
| **9** | 2-(4-hexyl-1H-1,2,3-triazol-1-yl)-2,2-diphenyl-1-(1H-tetrazol-5-yl)ethan-1-ol | ¹H NMR (300 MHz, CDCl₃) δ 7.21-7.38 (m, 10H), 6.82-6.93 (m, 3H), 2.63-2.65 (m, 1H), 1.57 (m, 2H), 1.26 (b, 6H), 0.85 (t, *J* = 5.8 Hz, 3H). ¹³C NMR (75 MHz, CDCl₃) δ 162.8, 139.2, 138.4, 129.3, 128.7, 128.4, 127.8, 124.2, 71.4, 31.3, 28.9, 28.7, 25.2, 22.4, 13.9. HRMS (ESI, TOF MS) m/z calculated for C₂₃H₂₈N₇O [M+H]⁺: 418.2355, found: 418.2353. | 60% (a) |
| **10** | 2-(4-(3-chloropropyl)-1H-1,2,3-triazol-1-yl)-2,2-diphenyl-1-(1H-tetrazol-5-yl)ethan-1-ol | Mp: 114-116°C. ¹H NMR (300 MHz, CDCl₃) δ 7.25-7.39 (m, 12H), 7.02 (bs, 1H), 6.83 (b, 4H), 3.55 (m, 2H), 2.82 (m, 2H), 2.11 (m, 2H). ¹³C NMR (75 MHz, CDCl₃) δ 139.1, 138.3, 129.5, 128.8, 128.5, 127.8, 125.2, 71.2, 43.9, 31.4, 22.4. HRMS (ESI, TOF MS) m/z calculated for C₂₀H₂₁N₇OCl [M+H]⁺: 410.1496, found: 410.1490. | 78% (a) |
| **11** | tert-butyl 1-(2-hydroxy-1,1-diphenyl-2-(1H-tetrazol-5-yl)ethyl)-1H-1,2,3-triazole-4-carboxylate | Mp: 122-124°C. ¹H NMR (300 MHz, CDCl₃) δ 7.69 (s, 1H), 7.43 (b, 5H), 7.27 (b, 3H), 6.94-6.97 (m, 3H), 1.59 (s, 9H). ¹³C NMR (75 MHz, CDCl₃) δ 159.2, 155.5, 139.7, 138.1, 130.0, 129.5, 128.9, 128.8, 128.5, 127.6, 83.1, 70.7, 27.9. HRMS (ESI, TOF MS) m/z calculated for C₂₂H₂₃N₇ONa [M+Na]⁺: 456.1760, found: 456.1754. | 49% (a) |

These results evidence that the applicant successfully conceived and achieved a CuAAC reaction between the alpha-hydroxy-beta-azido-tetrazoles and terminal alkynes, to manufacture alpha-hydroxy-beta-triazolo-tetrazoles.

Procedures (a) and (b) are thus efficient methods to prepare a wide range of alpha-hydroxy-beta-triazolo-tetrazoles compounds.

### Example 3: Triazole alkynes

Hereafter are provided triazoles alkynes according to the invention. R¹, R² and R³ groups refer to formula (III):

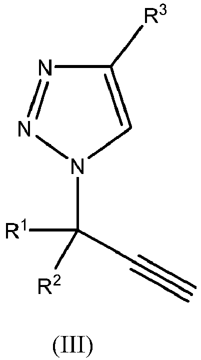

| **#** | **R¹** | **R²** | **R³** | **Formula** | **Name** |
|---|---|---|---|---|---|
| **12** | pentyl (cyclohexyl) | | Ph | 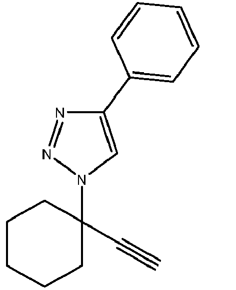 | 1-(1-ethynylcyclohexyl) -4-phenyl-1H-1,2,3-triazole |
| **13** | hexyl (cycloheptyl) | | Ph | 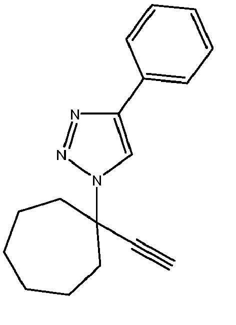 | 1-(1-ethynylcycloheptyl )-4-phenyl-1H-1,2,3-triazole |
| **14** | heptyl (cyclooctyl) | | Ph | 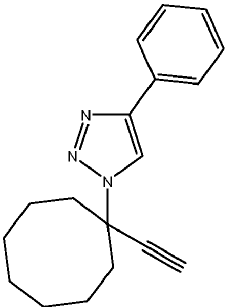 | 1-(1-ethynylcyclooctyl)-4-phenyl-1H-1,2,3-triazole |
| **15** | heptyl (cyclooctyl) | | tBuAc | 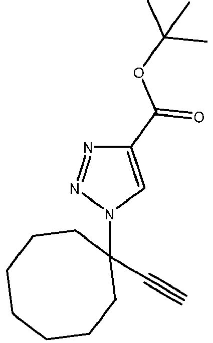 | tert-butyl 1-(1-ethynylcyclooctyl)-1H-1,2,3-triazole-4-carboxylate |
| **16** | heptyl (cyclooctyl) | | 3-chloroprop yl | 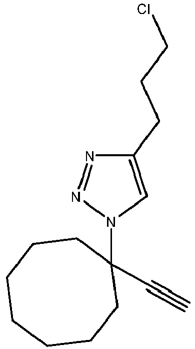 | 4-(3-chloropropyl)-1-(1-ethynylcyclooctyl)-1H-1,2,3-triazole |
| **17** | heptyl (cyclooctyl) | | 2-hydroxy ethyl | 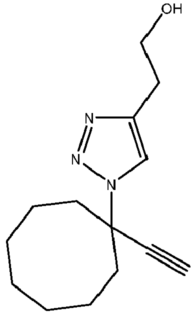 | 2-(1-(1-ethynylcyclooctyl)-1H-1,2,3-triazol-4-yl)ethan-1-ol |
| **18** | Et | Et | Ph | 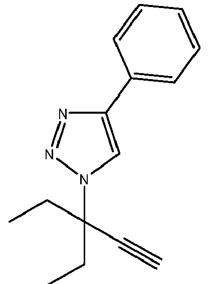 | 1-(3-ethylpent-1-yn-3-yl)-4-phenyl-1H-1,2,3-triazole |
| **19** | Ph | H | hexyl | 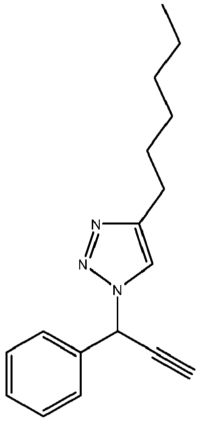 | 4-hexyl-1-(1-phenylprop-2-yn-1-yl)-1H-1,2,3-triazole |
| **20** | Ph | Ph | hexyl | 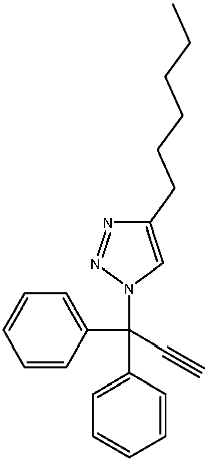 | 1-(1,1-diphenylprop-2-yn-1-yl)-4-hexyl-1H-1,2,3-triazole |

These compounds may be prepared as disclosed in Example 4.

### Example 4: Synthesis of triazole alkynes

### Materials and Methods

### Procedures for the reaction of alpha-hydroxy-beta-triazolo-tetrazoles with carbodiimides

Procedure (c), with diisopropylcarbodiimide (DIC): The alpha-hydroxy-beta-triazolotetrazole (0.35 mmol) was dissolved in dichloromethane (10 mL). DIC (0.42 mmol) was added. The mixture was stirred at room temperature for 18 hours, and then concentrated under reduced pressure. The residue was purified by flash chromatography on silica gel.

Procedure (d), with N-(3-dimethylaminopropyl)-N'-ethylcarbodiimide (EDC): The alpha -hydroxy- beta-triazolo-tetrazole (0.1 mmol) was dissolved in dichloromethane (5 mL). EDC (0.12 mmol) was added and the mixture was stirred at room temperature for 18 hours. The mixture was diluted with dichloromethane and the resulting solution was washed successively with solutions of aqueous 0.5M HCl, aqueous saturated NaCl and aqueous saturated NaHCO₃. The organic layer was dried over MgSO₄ and concentrated under reduced pressure. The residue was purified by flash chromatography on silica gel.

### Results

Hereafter are provided the yield and physical characterization of triazole alkynes according to the invention, prepared by procedures (c) and (d).

Compounds #12-20 were prepared from the corresponding alpha-hydroxy-beta-triazolo-tetrazoles disclosed in Example 1 and 2.

| **#** | **Name** | **Characterization** | **Yield** |
|---|---|---|---|
| **12** | 1-(1-ethynylcyclohexyl )-4-phenyl-1H-1,2,3-triazole | Mp: 114-116°C. ¹H NMR (300 MHz, CDCl₃) δ 8.18 (d, *J* =4.6 Hz, 1H), 7.85-7.89 (m, 2H), 7.26-7.47 (m, 3H), 2.78 (d, *J* = 4.7 Hz, 1H), 2.25-2.43 (m, 4H), 1.77-1.88 (m, 5H), 1.28-1.43 (m, 1H). ¹³C NMR (75 MHz, CDCl₃) δ 146.7, 130.7, 128.7, 128.0, 125.7, 118.5, 82.4, 76.2, 61.5, 38.6, 24.7, 23.1. HRMS (ESI, TOF MS) m/z calculated for C₁₆H₁₈N₃ [M+H]⁺: 252.1501, found: 252.1498. | 69% (c) |
| **13** | 1-(1-ethynylcyclohepty 1)-4-phenyl-1H-1,2,3-triazole | Mp: 80-81°C. ¹H NMR (300 MHz, CDCl₃) δ 8.18 (s, 1H), 7.85 (m, 2H), 7.40-7.45 (m, 2H), 7.33 (m, 1H), 2.79 (s, 1H), 2.59-2.63 (m, 2H), 2.27-2.34 (m, 2H), 1.66-1.87 (m, 8H). ¹³C NMR (75 MHz, CDCl₃) δ 146.5, 130.6, 128.7, 128.0, 125.7, 118.5, 83.7, 75.4, 64.6, 42.1, 27.9, 22.7. HRMS (ESI, TOF MS) m/z calculated for C₁₇H₂₀N₃ [M+H]⁺: 266.1657, found: 266.1655. | 61% (c) |
| **14** | 1-(1-ethynylcyclooctyl )-4-phenyl-lH-1,2,3-triazole | Mp: 78°C. ¹H NMR (300 MHz, CDCl₃) δ 8.15 (s, 1H), 7.85 (m, 2H), 7.40-7.45 (m, 2H), 7.33 (t, *J* = 7.2 Hz, 1H), 2.72 (s, 1H), 2.64-2.71 (m, 2H), 2.24-2.33 (m, 2H), 1.63-1.81 (m, 12H). ¹³C NMR (75 MHz, CDCl₃) δ 146.7, 130.6, 128.7, 128.0, 125.7, 118.5, 83.9, 74.7, 64.3, 37.0, 27.7, 24.4, 22.4. HRMS (ESI, TOF MS) m/z calculated for C₁₈H₂₂N₃ [M+H]⁺: 280.1814, found: 280.1817 | 73% (c) |
| **15** | tert-butyl 1-(1-ethynylcyclooctyl )-1H-1,2,3-triazole-4-carboxylate | ¹H NMR (300 MHz, CDCl₃) δ 8.35 (s, 1H), 2.73 (s, 1H), 2.57-2.62 (m, 2H), 2.18-2.24 (m, 2H), 1.61-1.75 (m, 21H). ¹³C NMR (75 MHz, CDCl₃) δ 160.2, 140.5, 130.6, 125.9, 83.1, 82.2, 75.2, 64.8, 37.0, 28.2, 27.6, 24.3, 22.3. HRMS (ESI, TOF MS) m/z calculated for C₁₇H₂₆N₃O₂ [M+H]⁺: 304.2025, found: 304.2031. | 76% (d) |
| **16** | 4-(3-chloropropyl)-1-(1-ethynylcyclooctyl )-1H-1,2,3-triazole | ¹H NMR (300 MHz, CDCl₃) δ 7.69 (s, 1H), 3.59 (t, *J* = 6.4 Hz, 2H), 2.89 (t, *J* =7.7 Hz, 2H), 2.67 (s, 1H), 2.57-2.64 (m, 2H), 2.16-2.26 (m, 4H), 1.62-1.76 (m, 10H). ¹³C NMR (75 MHz, CDCl₃) δ 145.6, 119.9, 83.9, 74.4, 63.9, 44.3, 36.9, 31.9, 27.7, 24.3, 22.7, 22.4. HRMS (ESI, TOF MS) m/z calculated for C₁₅H₂₃N₃Cl [M+H]⁺: 280.1581, found: 280.1587. | 75% (d) |
| **17** | 2-(1-(1-ethynylcyclooctyl )-1H-1,2,3-triazol-4-yl)ethan-1-ol | ¹H NMR (300 MHz, CDCl₃) δ 7.76 (s, 1H), 3.95 (t, *J =* 5.6 Hz, 1H), 2.86-2.98 (m, 3H), 2.67 (s, 1H), 2.55-2.64 (m, 2H), 2.17-2.25 (m, 2H), 1.61-1.76 (m, 10H). ¹³C NMR (75 MHz, CDCl₃) δ 144.5, 120.3, 83.8, 74.5, 64.0, 61.5, 36.9, 28.6, 27.7, 24.3, 22.4. HRMS (ESI, TOF MS) m/z calculated for C₁₄H₂₂N₃O [M+H]⁺: 248.1763, found: 248.1770. | 46% (d) |
| **18** | 1-(3-ethylpent-1-yn-3-yl)-4-phenyl-1H-1,2,3-triazole | Mp: 36-38°C. ¹H NMR (300 MHz, CDCl₃) δ 8.17 (s, 1H), 7.87-7.90 (m, 2H), 7.40-7.45 (m, 2H), 7.33 (m, 1H), 2.79 (s, 1H), 2.38-2.50 (m, 2H), 2.07-2.19 (m, 2H), 0.89 (t, *J* =7.4 Hz 6H). ¹³C NMR (75 MHz, CDCl₃) δ 146.0, 130.7, 128.7, 127.9, 125.6, 120.6, 81.2, 76.3, 66.0, 34.8, 8.5. HRMS (ESI, TOF MS) m/z calculated for C₁₅H₁₈N₃ [M+H]⁺: 240.1501, found: 240.1502 | 73% (c) |
| **19** | 4-hexyl-1-(1-phenylprop-2-yn-1-yl)-1H-1,2,3-triazole | Mp: 59-61°C. ¹H NMR (300 MHz, CDCl₃) δ 7.37-7.48 (m, 6H), 6.67 (d, *J* =2.0 Hz, 1H), 2.82 (d, *J* =2.4 Hz, 1H), 2.68 (t, *J* =7.5 Hz, 2H), 1.59-1.69 (m, 2H), 1.28-1.36 (m, 6H), 0.86 (t, *J* =7.0 Hz, 3H). ¹³C NMR (75 MHz, CDCl₃) δ 149.0, 135.6, 129.2, 128.9, 127.0, 119.0, 78.1, 55.5, 31.4, 29.2, 28.8, 25.7, 22.5, 13.9. HRMS (ESI, TOF MS) m/z calculated for C₁₇H₂₂N₃ [M+H]⁺: 268.1814, found: 268.1812. | 75% (c) |
| **20** | 1-(1,1-diphenylprop-2-yn-1-yl)-4-hexyl-1H-1,2,3-triazole | Mp: 59-61°C. ¹H NMR (300 MHz, CDCl₃) δ 7.25-7.44 (m, 11H), 3.09 (s, 1H), 2.74 (t, *J* =8.0 Hz, 1H), 1.63-1.70 (m, 2H), 1.29-1.33 (m, 6H), 0.80 (b, 3H). ¹³C NMR (75 MHz, CDCl₃) δ 147.7, 140.3, 128.8, 128.4, 127.9, 121.6, 83.2, 77.6, 69.1, 31.5, 29.3, 28.9, 25.7, 22.5, 14.0. HRMS (ESI, TOF MS) m/z calculated for C₂₃H₂₆N₃ [M+H]⁺: 344.2127, found: 344.2132. | 62% (d) |

Surprisingly, the formation of a five-membered cycle or heterocycle was never observed by the applicant when carrying out the reaction of alpha-hydroxy-beta-triazolo-tetrazoles with carbodiimides. The triazoles alkyne is thus the only product of the reaction.

These results evidence that the alpha-hydroxy-beta-triazolo-tetrazoles according to the invention may be efficiency converted to triazoles alkynes, by procedures (c) and (d) according to the invention, which have a very broad scope while being very efficient.

### Example 5: Di-triazoles and synthesis thereof

### Compounds

Hereafter are provided di-triazoles according to the invention. R¹, R², R³ and R⁴ groups refer to formula (IV):

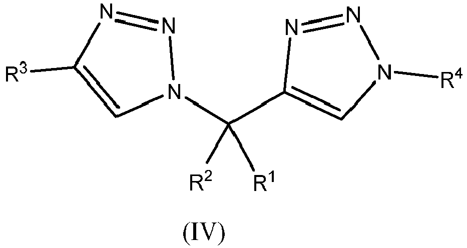

| **#** | **R¹** | **R²** | **R³** | **R⁴** | **Formula** | **Name** |
|---|---|---|---|---|---|---|
| **21** | pentyl (cyclo hexyl) | | Ph | octyl | 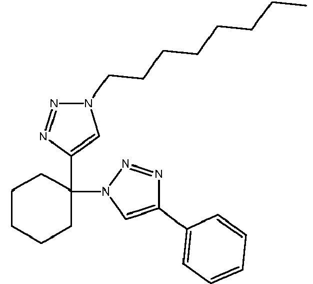 | 1-octyl-4-(1-(4-phenyl-1H-1,2,3-triazol-1-yl)cyclohexyl)-1H-1,2,3-triazole |
| **22** | pentyl (cyclo hexyl) | | Ph | bis(cyclo pentadien yl)iron | 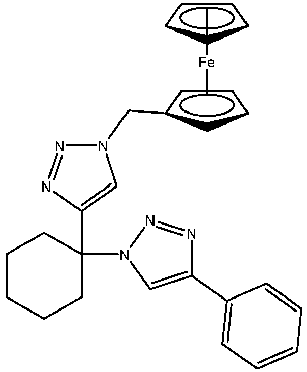 | 1-[bis(η5-cyclopentadienyl) iron]-4-(1-(4-phenyl-1H-1,2,3-triazol-1-yl)cyclohexyl)-1H-1,2,3-triazole |
| **23** | pentyl (cycl ohexyl) | | Ph | carbo hydrate | 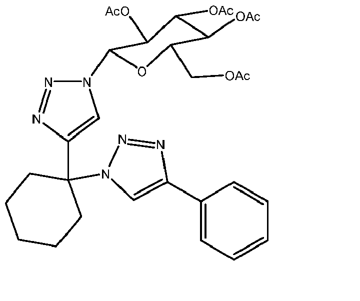 | (2S,3S,4R,5S,6S) -2-(acetoxymethyl)-6-(4-(1-(4-phenyl-1H-1,2,3-triazol-1-yl)cyclohexyl)-1H-1,2,3-triazol-1-yl)tetrahydro-2H-pyran-3,4,5-triyl triacetate |
| **24** | pentyl (cyclo hexyl) | | Ph | -CH₂CH (COOMe) NHBoc | 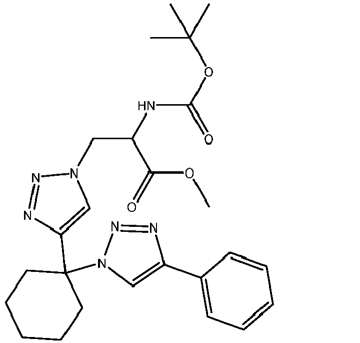 | methyl2-((tert-butoxycarbonyl)a mino)-3-(4-(1-(4-phenyl-1H-1,2,3-triazol-1-yl)cyclohexyl)-1H-1,2,3-triazol-1-yl)propanoate |
| **25** | heptyl (cyclo octyl) | | Ph | octyl | 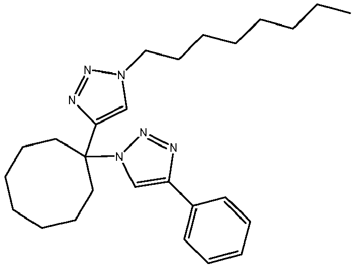 | 1-octyl-4-(1-(4-phenyl-1H-1,2,3-triazol-1-yl)cyclooctyl)-1H-1,2,3-triazole |
| **26** | heptyl (cyclo octyl) | | Ph | EtCOO-propyl | 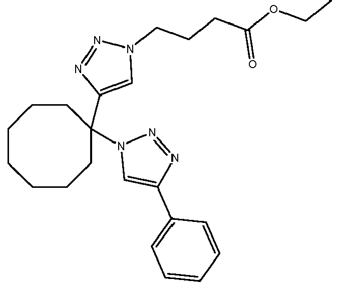 | ethyl 4-(4-(1-(4-phenyl-1H-1,2,3-triazol-1-yl)cyclooctyl)-1H-1,2,3-triazol-1-yl)butanoate |
| **27** | heptyl (cyclo octyl) | | Ph | pyren-1-ylmethyl | 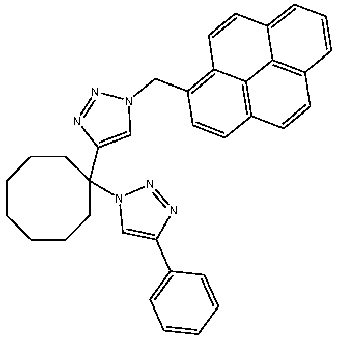 | 4-phenyl-1-(1-(1-(pyren-1-ylmethyl)-1H-1,2,3-triazol-4-yl)cyclooctyl)-1H-1,2,3-triazole |
| **28** | heptyl (cyclo octyl) | | Ph | benzyl | 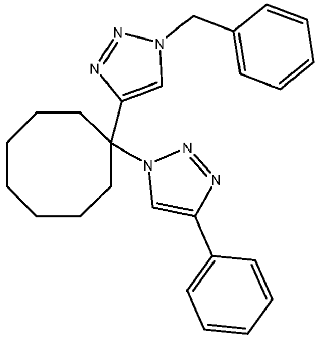 | 1-benzyl-4-(1-(4-phenyl-1H-1,2,3-triazol-1-yl)cyclooctyl)-1H-1,2,3-triazole |
| **29** | heptyl (cyclo octyl) | | Ph | -C(Et)₂-CH(OH)-Tz | 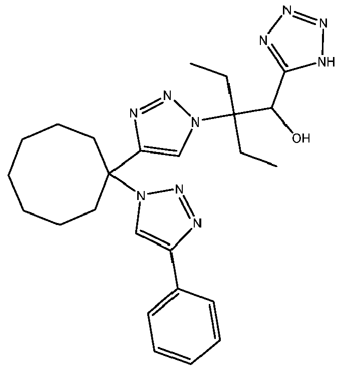 | 2-ethyl-2-(4-(1-(4-phenyl-1H-1,2,3-triazol-1-yl)cyclooctyl)-1H-1,2,3-triazol-1-yl)-1-(1H-tetrazol-5-yl)butan-1-ol |

### Materials and Methods

### Procedure (e) for the CuAAC reaction of triazole alkynes:

The triazole alkyne substrate (0.1 mmol) was dissolved in *n*-BuOH (1 mL). An azide (0.3 mmol) was added. A solution of sodium ascorbate (0.02 mmol in 0.25 mL water) was added, followed by a solution of copper sulphate (0.01 mmol in 0.25 mL water). The mixture was stirred at room temperature for 48 hours. The organic phase was separated and concentrated under reduced pressure. The residue was purified by flash chromatography on silica gel.

### Results

Hereafter are provided the yield and physical characterization of di-triazoles according to the invention, prepared by procedure (e).

Compounds #21-29 were prepared from the corresponding triazole alkynes disclosed in Examples 3 and 4 and from the appropriate R⁴-N₃ azide.

| **#** | **Name** | **Characterization** | **Yield** |
|---|---|---|---|
| **21** | 1-octyl-4-(1-(4-phenyl-1H-1,2,3-triazol-1-yl)cyclohexyl)-1H-1,2,3-triazole | Mp: 110-112°C. ¹H NMR δ 7.93 (s, 1H), 7.81-7.84 (m, 2H), 7.26-7.42 (m, 4H), 4.26 (t, *J* =6.9 Hz, 2H), 2.65-2.80 (m, 4H), 1.84 (b, 2H), 1.57-1.64 (m, 6H), 1.23-1.27 (m, 10H), 0.85 (t, *J* =6.1 Hz, 3H). ¹³C NMR (75 MHz, CDCl₃) δ 150.8, 147.2, 130.5, 128.7, 128.0, 125.6, 121.1, 118.3, 62.3, 50.5, 35.7, 31.6, 30.1, 28.9, 28.8, 26.4, 24.8, 22.5, 22.0, 14.0. HRMS (ESI, TOF MS) m/z calculated for C₂₄H₃₅N₆ [M+H]⁺: 407.2923, found: 407.2924. | 98% |
| **22** | 1-[bis(η5-cyclopentadienyl)ir on]-4-(1-(4-phenyl-1H-1,2,3-triazol-1-yl)cyclohexyl)-1H-1,2,3-triazole | Mp: 157-163°C (dec.). ¹H NMR δ 7.92 (s, 1H), 7.80-7.83 (m, 2H), 7.27-7.40 (m, 4H), 5.22 (bs, 2H), 4.14-4.24 (m, 9H), 2.65-2.75 (m, 4H), 1.84 (b, 2H), 1.58-1.62 (m, 6H). ¹³C NMR (75 MHz, CDCl₃) δ 150.8, 147.3, 130.6, 128.7, 127.9, 125.5, 120.6, 118.3, 80.4, 69.1, 68.8, 62.1, 50.1, 35.7, 24.8, 21.9. HRMS (ESI, TOF MS) m/z calculated for C₂₇H₂₈N₆Fe [M+H]⁺: 492.1724, found: 492.1729. | 75% |
| **23** | 2-(acetoxymethyl)-6-(4-(1-(4-phenyl-1H-1,2,3-triazol-1-yl)cyclohexyl)-1H-1,2,3-triazol-1-yl)tetrahydro-2H-pyran-3,4,5-triyl triacetate | ¹H NMR δ 7.86-7.84 (m, 3H), 7.66 (s, 1H), 7.30-7.42 (m, 3H), 5.82 (d, *J* =9.0 Hz, 1H), 5.23-5.39 (m, 2H), 5.33 (m, 1H), 4.27-4.33 (m, 1H), 4.11-4.15 (m, 1H), 3.99-4.02 (m, 1H), 2.70-2.81 (m, 4H), 2.07 (s, 3H), 2.05 (s, 3H), 2.01 (s, 3H), 1.84 (s, 3H), 1.57-1.62 (m, 6H). ¹³C NMR (75 MHz, CDCl₃) δ 170.4, 169.8, 169.2, 168.8, 130.6, 128.7, 128.0, 125.5, 120.5, 85.9, 75.2, 72.2, 70.6, 67.6, 62.3, 61.4, 36.0, 35.6, 24.8, 22.0, 21.9, 20.6, 20.4, 20.0. HRMS (ESI, TOF MS) m/z calculated for C₃₀H₃₇N₆O₉ [M+H]⁺: 625.2622, found: 625.2626. | 93% |
| **24** | methyl 2-((tert-butoxycarbonyl)am ino)-3-(4-(1-(4-phenyl-1H-1,2,3-triazol-1-yl)cyclohexyl)-1H-1,2,3-triazol-1-yl)propanoate | ¹H NMR δ 7.81-7.89 (m, 3H), 7.29-7.41 (m, 4H), 5.42 (d, *J* =6.9 Hz), 4.66-4.76 (m, 3H), 3.72 (s, 3H), 2.61-2.77 (m, 4H), 1.55-1.62 (m, 6H), 1.38 (s, 9H). ¹³C NMR (75 MHz, CDCl₃) δ 169.3, 154.9, 130.4, 128.7, 128.0, 125.6, 122.8, 118.4, 80.7, 62.2, 53.6, 53.0, 51.0, 35.8, 35.7, 28.1, 24.8, 22.0. HRMS (ESI, TOF MS) m/z calculated for C₂₅H₃₄N₇O₄ [M+H]⁺: 496.2672, found: 496.2669. | 89% |
| **25** | 1-octyl-4-(1-(4-phenyl-1H-1,2,3-triazol-1-yl)cyclooctyl)-1H-1,2,3-triazole | ¹H NMR δ 7.93 (s, 1H), 7.80-7.83 (m, 2H), 7.27-7.41 (m, 4H), 4.29 (t, *J* =6.9 Hz, 2H), 2.75-2.96 (m, 4H), 1.84-1.89 (m, 2H), 1.65 (b, 10H), 1.24-1.28 (m, 10H), 0.86 (t, *J* =6.1 Hz, 3H). ¹³C NMR (75 MHz, CDCl₃) δ 151.2, 130.7, 128.7, 127.9, 125.5, 121.3, 118.6, 66.1, 50.4, 33.5, 31.6, 30.0, 28.9, 28.8, 27.9, 26.4, 24.6, 22.5, 22.0, 14.0. HRMS (ESI, TOF MS) m/z calculated for C₂₆H₃₈N₆Na [M+Na]⁺: 457.3056, found: 457.3061. | 92% |
| **26** | ethyl 4-(4-(1-(4-phenyl-1H-1,2,3-triazol-1-yl)cyclooctyl)-1H-1,2,3-triazol-1-yl)butanoate | ¹H NMR δ 7.95 (s, 1H), 7.80-7.83 (m, 2H), 7.27-7.46 (m, 4H), 4.38 (t, *J* =6.7 Hz, 2H), 4.10 (m, 2H), 2.73-2.96 (m, 4H), 2.15-2.35 (m, 4H), 1.64 (b, 10H), 1.23 (m, *J* =6.5 Hz, 3H). ¹³C NMR (75 MHz, CDCl₃) δ 172.1, 151.2, 146.9, 130.6, 128.7, 127.9, 125.5, 121.7, 118.7, 66.1, 60.7, 49.4, 33.5, 30.7, 27.9, 25.2, 24.6, 21.9, 14.1. HRMS (ESI, TOF MS) m/z calculated for C₂₄H₃₂N₆O₂Na [M+Na]⁺: 459.2484, found: 459.2479. | 83% |
| **27** | 4-phenyl-1-(1-(1-(pyren-1-ylmethyl)-1H-1,2,3-triazol-4-yl)cyclooctyl)-1H-1,2,3-triazole | H NMR δ 8.02-8.25 (m, 8H), 7.73-7.94 (m, 4H), 7.27-7.36 (m, 4H), 6.20 (bs, 2H), 2.62-2.85 (m, 4H), 1.55 (b, 10H). ¹³C NMR (75 MHz, CDCl₃) δ 151.3, 132.2, 131.1, 130.5, 129.0, 128.7, 128.3, 128.0, 127.7, 127.2, 126.4, 126.3, 125.9, 125.8, 125.6, 125.0, 124.9, 124.4, 121.7, 121.6, 118.7, 66.3, 52.6, 33.3, 27.8, 24.6, 21.9. HRMS (ESI, TOF MS) m/z calculated for C₃₅H₃₂N₆Na [M+Na]⁺: 559.2586, found: 559.2584. | 100% |
| **28** | 1-benzyl-4-(1-(4-phenyl-1H-1,2,3-triazol-1-yl)cyclooctyl)-1H-1,2,3-triazole | Mp: 133-134°C. ¹H NMR δ 7.97 (s, 1H), 7.82-7.85 (m, 2H), 7.27-7.41 (m, 9H), 5.50 (bs, 2H), 2.73-2.95 (m, 4H), 1.65 (b, 10H). ¹³C NMR (75 MHz, CDCl₃) δ 151.5, 146.9, 134.1, 130.5, 129.1, 128.7; 128.0, 127.9, 125.5, 121.5, 118.6,66.1,54.2,33.4,27.9,24.6,21.9. HRMS (ESI, TOF MS) m/z calculated for C₂₅H₂₉N₆ [M+H]⁺: 413.2454, found: 413.2464 | 90% |
| **29** | 2-ethyl-2-(4-(1-(4-phenyl-1H-1,2,3-triazol-1-yl)cyclooctyl)-1H-1,2,3-triazol-1-yl)-1-(1H-tetrazol-5-yl)butan-1-ol | ¹H NMR δ 8.59 (s, 1H), 8.33, (s, 1H), 8.18-8.21 (m, 2H), 7.32-7.44 (m, 3H), 2.54-3.05 (m, 8H), 1.41-1.59 (b, 10H), 0.94-1.03 (m, 6H). ¹³C NMR (75 MHz, pyridine-d₅) δ 151.2, 130.7, 128.7, 127.9, 125.5, 121.3, 118.6, 66.1, 50.4, 33.5, 31.6, 30.0, 28.9, 28.8, 27.9, 26.4, 24.6, 22.5, 22.0, 14.0. HRMS (ESI, TOF MS) m/z calculated for C₂₅H₃₅N₁₀O [M+H]⁺: 491.2995, found: 491.2996. | 91% |

These results evidence that the triazoles alkynes according to the invention may be efficiency reacted with azides in CuAAC conditions to prepare various di-triazoles compounds.

### Example 6: Poly-triazoles and synthesis thereof

### Compounds

Hereafter are provided poly-triazoles according to the invention.

| **#** | **Formula** | **Name** |
|---|---|---|
| **30** | 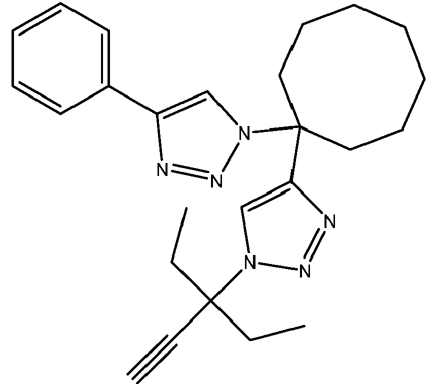 | 1-(3-ethylpent-1-yn-3-yl)-4-(1-(4-phenyl-1H-1,2,3-triazol-1-yl)cyclooctyl)-1H-1,2,3-triazole |
| **31** | 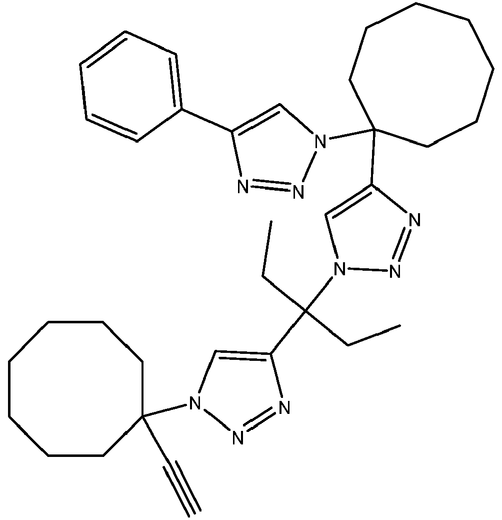 | 1-(-ethynylcyclooctyl)-4-(3-(4-(1-(4-phenyl-1H-1,2,3-triazol-1-yl)cyclooctyl)-1H-1,2,3-triazol-1-yl)pentan-3-yl)-1H-1,2,3-triazole |
| **32** | 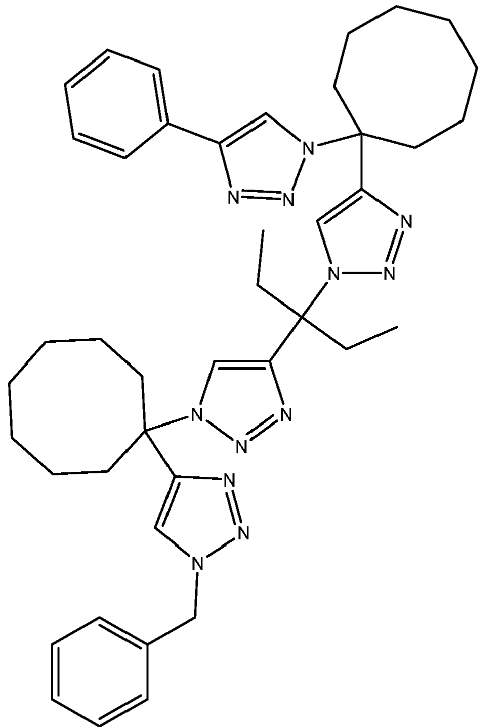 | 1-benzyl-4-(1-(4-(3-(4-(1-(4-phenyl-1H-1,2,3-triazol-1-yl)cyclooctyl)-1H-1,2,3-triazol-1-yl)pentan-3-yl)-1H-1,2,3-triazol-1-yl)cyclooctyl)-1H-1,2,3-triazole |

### Materials and Methods

### Procedure for synthesis of bis-triazole alkyne #30:

2-Azido-2-ethyl-1-(1H-tetrazol-5-yl)-butan-1-ol (28mg, 0.18 mmol) and 1-(1-ethynylcyclooctyl)-4-phenyl-1H-[1,2,3]triazole (45mg, 0.16 mmol) #14 were dissolved in nBuOH (2 mL). TBTA (tris((1-benzyl-1H-1,2,3-triazolyl)methyl)amine) (10.5mg, 0.02 mmol) was added. A solution of sodium ascorbate (11mg, 0.05 mmol in 0.5 mL water) was added, followed by a solution of copper sulphate (5mg, 0.02 mmol in 0.5 mL water). The mixture was stirred at room temperature for 48 hours. The mixture was concentrated under reduced pressure.

The residue was dissolved in 1,2-dichloroethane (3 mL). DIC (0.035mL, 0.22 mmol) was added. The mixture was stirred at 50°C for 3 hours, and then concentrated under reduced pressure. The residue was purified by flash chromatography on silica gel using petroleum ether/EtOAc: 90/10 as eluent (Rf = 0.18). The bis-triazole alkyne was isolated as a white solid (41 mg, 62 % yield).

### Procedure for synthesis of tris-triazole alkyne #31:

(1-Azido-cyclooctyl)-(1H-tetrazol-5-yl)-methanol (30 mg, 0.12 mmol) and the bis-triazole alkyne #30 (41mg, 0.1mmol) were dissolved in *n*-BuOH (2 mL) and THF (1mL). TBTA (tris((1-benzyl-1H-1,2,3-triazolyl)methyl)amine) (8mg, 0.015 mmol) was added. A solution of sodium ascorbate (7mg, 0.035 mmol in 0.5 mL water) was added, followed by a solution of copper sulphate (3mg, 0.012 mmol in 0.5 mL water). The mixture was stirred at room temperature for 5 days. The mixture was concentrated under reduced pressure.

The residue was dissolved in 1,2-dichloroethane (2 mL). DIC (0.02mL, 0.14 mmol) was added. The mixture was stirred at room temperature for 4 hours, and then concentrated under reduced pressure. The residue was purified by flash chromatography on silica gel using petroleum ether/EtOAc: 75/25 as eluent (Rf = 0.27). The tris-triazole alkyne was isolated as a white solid (31 mg, 52 % yield).

### Procedure for synthesis of tetra-triazole #32:

The tris-triazole alkyne **#31** (19mg, 0.032 mmol) was dissolved in *n*-BuOH (1 mL). Benzyl azide (0.01mL, 0.085 mmol) was added. A solution of sodium ascorbate (3mg, 0.015 mmol in 0.25 mL water) was added, followed by a solution of copper sulphate (1.2mg, 0.005 mmol in 0.25 mL water). The mixture was stirred at room temperature for 24 hours. The mixture was concentrated under reduced pressure. The residue was purified by flash chromatography on silica gel using dichloromethane/MeOH: 98/2 as eluent (Rf = 0.34). The tetra-triazole was isolated as white foam (20 mg, 86 % yield).

### Results

Hereafter are provided the yield and physical characterization of poly-triazoles according to the invention, prepared by the above procedures.

| **#** | **Name** | **Characterization** | **Yield** |
|---|---|---|---|
| **30** | 1-(3-ethylpent-1-yn-3-yl)-4-(1-(4-phenyl-1H-1,2,3-triazol-1-yl)cyclooctyl)-1H-1,2,3-triazole | ¹H NMR (300 MHz, CDCl₃) δ 7.90 (s, 1H), 7.80-7.83 (m, 3H), 7.27-7.42 (m, 3H), 2.92-3.00 (m, 2H), 2.73-2.80 (m, 2H), 2.72 (s, 1H), 2.28-2.40 (m, 2H), 2.03-2.14 (m, 2H), 1.66 (b, 10H), 0.81 (m, 6H). ¹³C NMR (75 MHz, CDCl₃) δ 149.3, 146.9, 130.7, 128.7, 127.9, 125.5, 122.6, 118.5, 80.7, 76.7, 66.2, 66.1, 34.7, 33.5, 27.9, 24.7, 22.0, 8.5. HRMS (ESI, TOF MS) m/z calculated for C₂₅H₃₂N₆Na [M+Na]⁺: 439.2586, found: 439.2585. | 62% |
| **31** | 1-(1-ethynylcyclooctyl )-4-(3-(4-(1-(4-phenyl-1H-1,2,3-triazol-1-yl)cyclooctyl)-1H-1,2,3-triazol-1-yl)pentan-3-yl)-1H-1,2,3-triazole | ¹H NMR (300 MHz, CDCl₃) δ 7.91 (s, 1H), 7.79-7.82 (m, 3H), 7.57 (s, 1H), 7.27-7.42 (m, 3H), 2.89-2.97 (m, 2H), 2.71-2.77 (m, 2H), 2.65 (s, 1H), 2.44-2.58 (m, 6H), 2.15-2.22 (m, 2H), 1.61-1.74 (m, 20H), 0.76 (m, 6H). ¹³C NMR (75 MHz, CDCl₃) δ 150.2, 147.9, 146.9, 130.6, 128.7, 127.9, 125.6, 121.0, 121.0, 118.7, 83.4, 75.0, 67.0, 66.2, 64.4, 37.0, 33.5, 30.3, 27.9, 27.6, 24.7, 24.3, 22.4, 22.0, 7.8. HRMS (ESI, TOF MS) m/z calculated for C₃₅H₄₇N₉Na [M+Na]⁺: 616.3852, found: 616.3859. | 52% |
| **32** | 1-benzyl-4-(1-(4-(3-(4-(1-(4-phenyl-1H-1,2,3-triazol-1-yl)cyclooctyl)-1H-1,2,3-triazol-1-yl)pentan-3-yl)-1H-1,2,3-triazol-1-yl)cyclooctyl)-1H-1,2,3-triazole | ¹H NMR (300 MHz, CDCl₃) δ 7.91 (s, 1H), 7.80-7.82 (m, 2H), 7.57 (s, 1H), 7.51 (s, 1H), 7.21-7.41 (m, 9H), 5.47 (s, 2H), 2.60-2.92 (m, 8H), 2.38-2.51 (m, 4H), 1.52-1.60 (m, 20H), 0.68-0.72 (m, 6H). ¹³C NMR (75 MHz, CDCl₃) δ 151.1, 148.1, 142.4, 142.2, 134.2, 130.5, 129.1, 128.8, 128.7, 128.0, 125.6, 121.5, 121.3, 121.2, 118.9, 118.7, 67.0, 66.4, 54.2, 33.6, 33.5, 30.3, 27.9, 27.8, 24.7, 24.6, 22.0, 21.9, 7.8. HRMS (ESI, TOF MS) m/z calculated for C₄₂H₅₅N₁₂ [M+H]⁺: 727.4673, found: 727.4678. | 86% |

These results evidence that successive iterations of the reactions according to the invention may be used to efficiently prepare poly-triazoles compounds substituted by different alkyl and aryl groups.

## Claims

1. An alpha-hydroxy-beta-triazolo-tetrazole compound of formula (I): 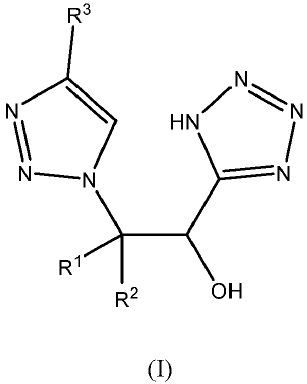 wherein R¹ and R² are each independently hydrogen, hydrocarbyl, aryl, heteroaryl, hydrocarbylaryl, arylhydrocarbyl, hydrocarbylheteroaryl, or heteroarylhydrocarbyl; or R¹ and R² form together a group being hydrocarbyl, aryl, heteroaryl, hydrocarbylaryl, arylhydrocarbyl, hydrocarbylheteroaryl, or heteroarylhydrocarbyl;
wherein the group is optionally substituted by at least one group being hydrocarbyl, aryl, heteroaryl, oxo, hydroxyl, amido, amino, nitro, carboxylo, formyl, halo, thioxo or sulfhydryl;
wherein the group is optionally interrupted or terminated by at least one group being -O-; -S-; or -NR^{N}- wherein R^{N} is hydrogen, hydrocarbyl, aryl, or a combination thereof;
wherein the nitrogen or sulfur atoms substituting or comprised in the group are optionally oxidized; and
wherein R³ is hydrogen, an organic group or an organic molecule;
and stereoisomers thereof; and salts thereof; and solvates thereof.

2. The compound according to claim **1,** wherein R¹ and R² are each independently hydrogen, alkyl, alkenyl, aryl, heteroaryl, alkylaryl, arylalkyl, alkenylaryl, or arylalkenyl groups; or wherein R¹ and R² form together a group being alkyl, alkenyl, aryl, alkylaryl, arylalkyl, alkenylaryl or arylalkenyl;
wherein the group is optionally substituted by at least one group being hydroxyl, alkyl, alkenyl, aryl, alkylaryl, arylalkyl, amino, nitro, halo or sulfhydryl; and
wherein the group is optionally interrupted or terminated by at least one group being -O-; -S-; and -NR^{N}- with wherein R^{N} is hydrogen, alkyl, alkenyl, aryl, or a combination thereof.

3. The compound according to claim **2,** wherein R¹ and R² are each independently hydrogen, alkyl, aryl or alkenylaryl groups or wherein R¹ and R² form together a group being alkyl or aryl;
wherein the group is optionally substituted by at least one halo group.

4. The compound according to anyone of claims **1** to **3,** wherein R³ is hydrogen, hydroxyl, amido, amino, cyano, nitro, tetrazolyl, triazolyl, borono, carboxylo, formyl, halo, haloformyl, phosphono, phosphate or sulfhydryl; or wherein R³ is a hydrocarbyl, aryl, heteroaryl, hydrocarbylaryl, arylhydrocarbyl, hydrocarbylheteroaryl, or heteroarylhydrocarbyl group;
wherein the group is optionally substituted by at least one group being hydrocarbyl, aryl, heteroaryl, oxo, hydroxyl, amido, amino, cyano, tetrazolyl, triazolyl, nitro, carboxylo, formyl, halo, thioxo or sulfhydryl;
wherein the group is optionally interrupted or terminated by at least one group being -O-; -S-; or -NR^{N}- wherein R^{N} is hydrogen, hydrocarbyl, aryl or a combination thereof; and
wherein the nitrogen or sulfur atoms substituting or comprised in the group are optionally oxidized;
or wherein R³ is a carbohydrate, an amino acid, a peptide or a nucleoside.

5. The compound according to anyone of claim **4,** wherein R³ is an alkyl, alkenyl, aryl, heteroaryl, alkylaryl, arylalkyl, alkenylaryl, arylalkenyl, alkylheteroaryl, or heteroarylalkyl group;
wherein the group is optionally substituted by at least one group being alkyl, alkenyl, aryl, heteroaryl, alkylaryl, arylalkyl, oxo, hydroxyl, amido, amino, tetrazolyl, triazolyl, nitro, carboxylo, formyl, halo, thioxo or sulfhydryl; and
wherein the group is optionally interrupted or terminated by at least one group being -O-; -S-; or -NR^{N}- wherein R^{N} is hydrogen, alkyl, alkenyl, aryl, or a combination thereof.

6. The compound according to anyone of claims **1** to **5,** being selected from the group consisting of: (1-(4-phenyl-1H-1,2,3-triazol-1-yl)cyclohexyl)(1H-tetrazol-5-yl)methanol; (1-(4-phenyl-1H-1,2,3-triazol-1-yl)cycloheptyl)(1H-tetrazol-5-yl)methanol; (1-(4-phenyl-1H-1,2,3-triazol-1-yl)cyclooctyl)(1H-tetrazol-5-yl)methanol; tert-butyl 1-(1-(hydroxy(1H-tetrazol-5-yl)methyl)cyclooctyl)-1H-1,2,3-triazole-4-carboxylate; (1-(4-(3-chloropropyl)-1H-1,2,3-triazol-1-yl)cyclooctyl)(1H-tetrazol-5-yl)methanol; 2-(1-(1-(hydroxy(1H-tetrazol-5-yl)methyl)cyclooctyl)-1H-1,2,3-triazol-4-yl)ethan-1-ol; 2-ethyl-2-(4-phenyl-1H-1,2,3-triazol-1-yl)-1-(1H-tetrazol-5-yl)butan-1-ol; 2-(4-hexyl-1H-1,2,3-triazol-1-yl)-2-phenyl-1-(1H-tetrazol-5-yl)ethan-1-ol; 2-(4-hexyl-1H-1,2,3-triazol-1-yl)-2,2-diphenyl-1-(1H-tetrazol-5-yl)ethan-1-ol; 2-(4-(3-chloropropyl)-1H-1,2,3-triazol-1-yl)-2,2-diphenyl-1-(1H-tetrazol-5-yl)ethan-1-ol; and tert-butyl 1-(2-hydroxy-1,1-diphenyl-2-(1H-tetrazol-5-yl)ethyl)-1H-1,2,3-triazole-4-carboxylate.

7. A process for manufacturing an alpha-hydroxy-beta-triazolo-tetrazole of formula (I): 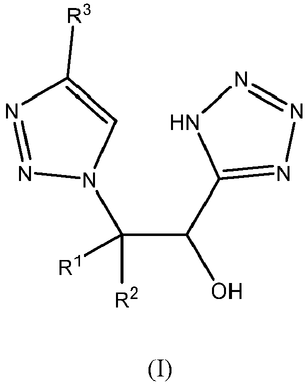 wherein R¹ and R² are each independently hydrogen, hydrocarbyl, aryl, heteroaryl, hydrocarbylaryl, arylhydrocarbyl, hydrocarbylheteroaryl, or heteroarylhydrocarbyl; or R¹ and R² form together a group being hydrocarbyl, aryl, heteroaryl, hydrocarbylaryl, arylhydrocarbyl, hydrocarbylheteroaryl, or heteroarylhydrocarbyl;
wherein the group is optionally substituted by at least one group being hydrocarbyl, aryl, heteroaryl, oxo, hydroxyl, amido, amino, nitro, carboxylo, formyl, halo, thioxo or sulfhydryl;
wherein the group is optionally interrupted or terminated by at least one group being -O-; -S-; or -NR^{N}- wherein R^{N} is hydrogen, hydrocarbyl, aryl, or a combination thereof; and
wherein the nitrogen or sulfur atoms are optionally oxidized; and
wherein R³ is hydrogen, an organic group or an organic molecule;
comprising starting from an alpha hydroxy-beta azido-tetrazole of formula (II): 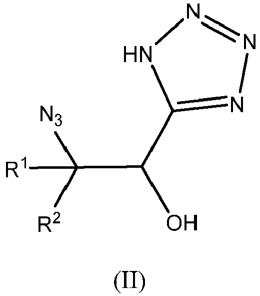 and a terminal alkyne of formula R³-C=C-H, and
carrying out the reaction of compound (II) with alkyne R³-C=C-H in the presence of a copper(I) source and a tertiary amine,
wherein the copper(I) source is either:
- a combination of a copper(I) salt and a base, or
- a combination of a copper(II) salt and a reducing agent.

8. The process according to claim **7,** wherein the copper(I) source is a combination of a copper(I) salt being copper(I) chloride, copper(I) bromide or copper(I) acetate; preferably copper(I) bromide; and a base being N,N-diisopropylethylamine.

9. The process according to claim **7,** wherein the copper(I) source is a combination of a copper(II) salt being copper(II) chloride, copper(II) bromide, copper(II) acetate or copper(II) sulphate; preferably copper(II) sulphate; and a reducing agent being sodium ascorbate or tri(2 carboxyethyl)phosphine; preferably sodium ascorbate; and
wherein the copper salt/reducing agent molar ratio in the reaction medium ranges from 0.1 to 2; preferably from 0.25 to 0.75; more preferably is 0.5.

10. The process according anyone of claims **7** to **9,** wherein the tertiary amine is tris(benzyltriazolylmethyl)amine, tris(tertbutyltriazolylmethyl)amine, tris(benzimidazole)methyl amine , 4,7-diphenyl-1,10-phenanthroline-disulfonic acid disodium salt, tris[2 (N,N dibenzylamino)ethyl] amine or tris(benzyltriazolylmethyl)amine; preferably tris(benzyltriazolylmethyl)amine.

11. The process according to anyone of claims **7** to **10,** wherein the alkyne/azide molar ratio in the reaction medium ranges from 1 to 10, preferably from 2 to 5, more preferably is 3; and wherein the copper salt/azide molar ratio in the reaction medium ranges from 0.01 to 2, preferably from 0.05 to 0.2, more preferably is 0.1.

12. The process according to anyone of claims **7** to **11,** wherein the copper salt/tertiary amine molar ratio in the reaction medium ranges from 0.1 to 10; preferably from 0.5 to 2; preferably is 1.

13. The process according to anyone of claims **7** to **12,** wherein the reaction is executed in a solvent; preferably ethanol, tetrahydrofuran, N,N'-dimethylformamide, acetonitrile, n-butyl alcohol, water or mixtures thereof; more preferably a mixture of n-butyl alcohol and water.

14. The process according to anyone of claims **7** to **13,** wherein the reaction is executed in a duration ranging from 12h to 5 days; preferably from 24h to 72h; more preferably for 48h.

15. The process according to anyone of claims **7** to **14**, wherein the reaction is executed at a temperature ranging from 0 to 50°C; preferably from 15 to 30°C; more preferably at 25°C.
